(19) Europäisches Patentamt
European Patent Office
Office européen des brevets

(11) EP 3 725 331 A1

(12) **EUROPEAN PATENT APPLICATION**
published in accordance with Art. 153(4) EPC

(43) Date of publication:
**21.10.2020 Bulletin 2020/43**

(21) Application number: **18887411.9**

(22) Date of filing: **30.11.2018**

(51) Int Cl.:
***A61K 47/02*** *(2006.01)* ***A61K 9/14*** *(2006.01)*
***A61K 31/4745*** *(2006.01)* ***A61K 47/44*** *(2017.01)*
***C01B 32/28*** *(2017.01)*

(86) International application number:
**PCT/JP2018/044300**

(87) International publication number:
**WO 2019/116936 (20.06.2019 Gazette 2019/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.12.2017 JP 2017241067**

(71) Applicants:
- **NATIONAL INSTITUTE OF ADVANCED INDUSTRIAL SCIENCE AND TECHNOLOGY**
  **Chiyoda-ku**
  **Tokyo 100-8921 (JP)**
- **Daicel Corporation**
  **Osaka 530-0011 (JP)**

(72) Inventors:
- **MIYAKO, Eijiro**
  **Tsukuba-shi, Ibaraki 305-8560 (JP)**
- **YU, Yue**
  **Tsukuba-shi, Ibaraki 305-8560 (JP)**
- **NISHIKAWA, Masahiro**
  **Tokyo 108-8230 (JP)**
- **LIU, Ming**
  **Tokyo 108-8230 (JP)**
- **TEI, Takahiro**
  **Tokyo 108-8230 (JP)**

(74) Representative: **Grünecker Patent- und Rechtsanwälte**
**PartG mbB**
**Leopoldstraße 4**
**80802 München (DE)**

(54) **MODIFIED CARBON NANOMATERIAL, NANOCLUSTER, SUBSTANCE DELIVERY CARRIER, AND PHARMACEUTICAL COMPOSITION**

(57) The present invention provides a self-assembled nanocluster containing a carbon nanomaterial modified with a functional group represented by the following formula (I): $-Y^1-R$ (I) wherein $Y^1$ represents a divalent linking group, and R represents an organic group.

Fig. 1

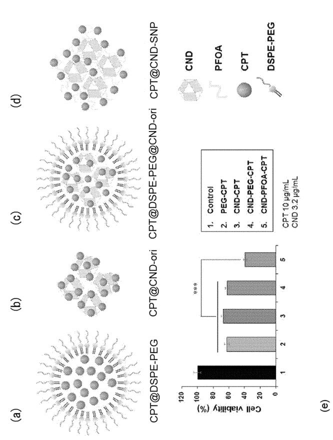


EP 3 725 331 A1

## Description

Technical Field

The present invention relates to a modified carbon nanomaterial, a nanocluster, a substance carrier, and a pharmaceutical composition.

In the present specification, the following abbreviations are used:

CND: Carbon Nano Diamond
SNP: Super Nano Particle

Background Art

Carbon nanodiamonds ("CNDs" below) have characteristics such as a small average particle size and large specific surface area, in addition to the inherent properties of diamonds. Additionally, CNDs have advantages in terms of their relatively low price and ease of availability. CNDs can be produced by a technique, such as an explosion method or a high-pressure high-temperature method (PTL 1). Due to their low toxicity, excellent biocompatibility, and stable fluorescence properties, applications of CNDs in biomedical fields have been widely studied. PTL 2 and 3 disclose modified CNDs, and production methods of modified CNDs.

For example of applications of CNDs in biomedical fields, NPL 1 reports that a cisplatin-CND composite was prepared by loading cisplatin, which is an anti-cancer agent, onto CNDs; that cisplatin can be released from the composite when the pH is within the acidic region; and that the drug released from the composite retains substantially the same cytotoxicity as that of free cisplatin. NPL 2 reports that an anti-cancer agent, epirubicin, was adsorbed to the surface of CNDS by hydrophobic interaction; and that the epirubicin-CND composite was wrapped with a lipid vesicle having an antibody specific to the epidermal growth factor receptor (EGFR) (anti-EGFR-PEG) of colorectal cancer bound thereto to increase the solubility. NPL 2 also reports the drug accumulation and efficacy of epirubicin against cancer cells.

Conventional drug-loaded CND composites have fundamental drawbacks in terms of a smaller amount of loaded drug and significantly decreased dispersibility in water after formation of the composites.

In order to increase the solubility, dispersibility, and dispersion stability of CNDs in water or polar organic solvents, modifying the surface of CNDs with a polymer has been suggested. For example, PTL 4 reports that modifying the surface of nanodiamonds with a specific group having a polyglycerol chain greatly increases the solubility, dispersibility, and dispersion stability in water or polar organic solvents. However, previous techniques have fundamentally focused on increasing the dispersibility of CNDs; there is no evaluation of self-assembling ability using chemically surface-modified CNDs, and no research on nanostructure control aimed at drug-loading.

Citation List

Patent Literature

PTL 1: JP2010-202458
PTL 2: JP2017-186234
PTL 3: JP2017-186235
PTL 4: JP2010-248023

Non-patent Literature

NPL 1: Bo Guan et al., Small 2010, 6, 1514-1519
NPL 2: Laura Moore et al., Adv. Mater. 2013, 25, 3532-3541

Summary of Invention

Technical Problem

A main object of the present invention is to provide a material that can be effectively loaded with a physiologically

active substance, such as a drug or flavor; and that exhibits excellent solubility in a physiological environment.

Solution to Problem

The present invention provides the following modified carbon nanomaterial, nanocluster, substance carrier, and pharmaceutical composition.

[1] A self-assembled nanocluster comprising a carbon nanomaterial modified with a functional group represented by the following formula (I):

$$-Y^1\text{-}R \qquad (I)$$

wherein $Y^1$ represents a divalent linking group, and R represents an organic group.

[2] The nanocluster according to [1], wherein the divalent linking group represented by $Y^1$ is one member selected from the group consisting of -NH-, -O-, -CO-O-, -O-CO-, -CO-NH-, -NH-CO-, -NH-CO-O-, -O-CO-NH-, -O-CO-O-, and -NH-CO-NH-.

[3] The nanocluster according to [1] or [2], wherein the organic group represented by R is a fluoroorganic group, a perfluoroorganic group, a substituted or unsubstituted alkyl group, a substituted or unsubstituted cycloalkyl group, a substituted or unsubstituted aryl group, a substituted or unsubstituted aralkyl group, or a substituted or unsubstituted heterocyclic group.

[4] The nanocluster according to any one of [1] to [3], wherein the carbon nanomaterial has at least one surface group selected from the group consisting of OH, COOH, and $NH_2$; and the functional group represented by formula (I) is incorporated in the carbon nanomaterial through the surface group.

[5] The nanocluster according to any one of [1] to [4], which is, together with an active ingredient, in the form of a composite.

[6] The nanocluster according to [5], wherein the active ingredient is a physiologically active substance, a labeling substance, a flavor, an essential oil, or an organic dye.

[7] The nanocluster according to any one of [1] to [6], wherein the carbon nanomaterial is a carbon nanodiamond or a carbon nanodot.

[8] A carrier for an active ingredient, the carrier comprising the nanocluster of any one of [1] to [7].

[9] A carbon nanomaterial modified with a functional group represented by the following formula (I):

$$-Y^1\text{-}R \qquad (I)$$

wherein $Y^1$ represents a divalent linking group, and R represents a fluoroorganic group or a perfluoroorganic group.

[10] The carbon nanomaterial according to [9], wherein the divalent linking group represented by $Y^1$ is one member selected from the group consisting of -NH-, -O-, -CO-O-, -O-CO-, -CO-NH-, -NH-CO-, -NH-CO-O-, -O-CO-NH-, -O-CO-O-, and -NH-CO-NH-.

[11] The carbon nanomaterial according to [9] or [10], which is a carbon nanodiamond.

[12] A pharmaceutical composition comprising the self-assembled nanocluster of [1], the self-assembled nanocluster, together with a drug, being in the form of a composite.

Advantageous Effects of Invention

The present invention can provide a cluster of a chemically surface-modified carbon nanomaterial that can be effectively loaded with an active ingredient, such as a physiologically active substance, a flavor, or an organic dye, and that exhibits excellent solubility in a physiological environment; and a composite material, such as a pharmaceutical

composition containing the nanocluster.

Brief Description of Drawings

Fig. 1 illustrates a cancer cell elimination effect of various nanomaterials that include the anti-cancer agent camptothecin (CPT). Fig. 1(a) illustrates a PEG-liposome that includes CPT (CPT@DSPE-PEG); Fig. 1(b) illustrates a composite of CPT and CND-ori (CPT@CND-ori); Fig. 1(c) illustrates a composite that contains CPT@CND-ori included in a liposome composed of DSPE-PEG (CPT@DSPE-PEG@CND-ori); Fig. 1(d) illustrates a composite of CPT and PFOA-modified CND (CPT@CND-SNP); and Fig. 1(e) illustrates the results of a cell viability test on U2OS cells using the materials of Figs. 1(a) to 1(d) and a control: 1. Control (a solvent alone), 2. PEG-CPT (CPT@DSPE-PEG), 3. CND-CPT (CPT@CND-ori), 4. CND-PEG-CPT (CPT@DSPE-PEG@CND-ori), and 5. CND-PFOA-CPT (CPT@CND-SNP).

Fig. 2 illustrates a synthesis method for a chemically surface-modified CND composite nanocluster (CND-SNP cluster) and characterization. Fig. 2(a) illustrates a synthesis method for a chemically surface-modified CND composite nanocluster (CND-SNP cluster). Fig. 2(b) illustrates a UV-VIS-NIR absorption spectrum of an aqueous solution of a chemically surface-modified CND composite nanocluster (CND-SNP cluster). Fig. 2(c) illustrates the results of particle size analysis of a chemically surface-modified CND composite nanocluster (CND-SNP cluster, average particle size: 90 nm) by dynamic light scattering. The photograph shows an aqueous solution of a chemically surface-modified CND composite nanocluster whose CND concentration was adjusted to 0.32 mg $mL^{-1}$. Fig. 2(d) illustrates structural analysis of a chemically surface-modified CND composite nanocluster (CND-SNP cluster) with a transmission electron microscope.

Fig. 3(a) illustrates thermogravimetric analysis of (1) CND before being subjected to chemical surface modification (CND-ori) and (2) a chemically surface-modified CND composite nanocluster ((1) CND-SNP cluster). Fig. 3(b) illustrates structural analysis of CND before being subjected to chemical surface modification (CND-ori) with a transmission electron microscope. Fig. 3(c) illustrates quantitative analysis (n = 3) of amino groups in CND before being subjected to chemical surface modification and a chemically surface-modified CND composite nanocluster by the Kaiser test. Fig. 3(d) illustrates the zeta potential (n = 3) of CND before being subjected to chemical surface modification (CND-ori) and a chemically surface-modified CND composite nanocluster (CND-SNP cluster). The CND concentration is 0.32 mg $mL^{-1}$. Fig. 3(e) illustrates the surface tension (n = 3) of CND before being subjected to chemical surface modification (CND-ori) and a chemically surface-modified CND composite nanocluster (CND-SNP cluster). The CND concentration is 0.32 mg $mL^{-1}$.

Fig. 4 illustrates intracellular distribution analysis, with a fluorescence microscope, of a chemically surface-modified CND composite nanocluster that includes the fluorescence probe BODIPY. Fig. 4(a) illustrates a method for producing a chemically surface-modified CND composite nanocluster that includes BODIPY. Fig. 4(b) illustrates a UV-VIS-NIR spectrum: the black line (1) indicates DMSO in which BODIPY was dissolved; the gray line (2) indicates an aqueous solution of a chemically surface-modified CND composite nanocluster (CND-SNP) before containing BODIPY; and the green line (3) indicates an aqueous solution of a chemically surface-modified CND composite nanocluster that includes BODIPY (BODIPY@CND-SNP). Fig. 4(c) illustrates fluorescence microscopic images of U2OS cells incubated at 4°C and 37°C for 2 hours with an aqueous solution of a chemically surface-modified CND composite nanocluster that included BODIPY. The CND concentration is 3.2 $\mu$g $mL^{-1}$. Fig. 4(d) illustrates Western blotting of LAMP1 (lysosome marker) in U2OS cells, in the presence or absence of a chemically surface-modified CND composite nanocluster. The CND concentration is 3.2 $\mu$g $mL^{-1}$. $\beta$-Tubulin was used as an introduction control. Fig. 4(e) illustrates fluorescence microscopic images of HeLa cells incubated at 37°C for 4 hours with an aqueous solution of a chemically surface-modified CND composite nanocluster that includes BODIPY (BODIPY@CND-SNP). Red indicates a lysosome, and green indicates a chemically surface-modified CND composite nanocluster that includes BODIPY.

Fig. 5 illustrates intracellular distribution behavior of a chemically surface-modified CND composite nanocluster. Fig. 5(a) illustrates fluorescence spectrum analysis of an aqueous solution of a chemically surface-modified CND composite nanocluster before and after containing the BODIPY fluorescence probe. The gray line (1) indicates before inclusion of BODIPY, and the green line (2) indicates after inclusion of BODIPY. Fig. 5(b) illustrates optical microscope analysis (bright-field image) of U2OS cells before and after a chemically surface-modified CND composite nanocluster (CND-SNP) was introduced into the cells. CND concentration: 3.2 $\mu$g $mL^{-1}$; incubation time: 12 hours. In Fig. 5(b), the upper-right arrow in CND-SNP indicates a chemically surface-modified CND composite nanocluster introduced into cells.

Fig. 6 illustrates a cancer cell elimination effect of a chemically surface-modified CND composite nanocluster that includes the anti-cancer agent camptothecin (CPT) *in vitro.* Fig. 6(a) illustrates a UV-VIS-NIR absorption spectrum

of an aqueous solution of a chemically surface-modified CND composite nanocluster before and after CPT was introduced (gray line (2): before CPT was included (CND-SNP), red line (3): after CPT was included (CPT@CND-SNP)), and (black line (1): DMSO in which CPT was dissolved). Fig. 6(b) illustrates evaluation of cytotoxicity of CPT and a chemically surface-modified CND composite nanocluster that includes CPT (CPT@CND-SNP) on U2OS cells; incubation time, 24 hours. Fig. 6(c) illustrates live cell staining using crystal violet of U2OS cells that were incubated with Control, CPT, CND-SNP, and a chemically surface-modified CND composite nanocluster that included CPT (CPT@CND-SNP) for 48 hours. Fig. 6(d) illustrates apoptosis analysis using flow cytometry of U2OS cells that were incubated with Control, CPT, CND-SNP, and a chemically surface-modified CND composite nanocluster that included CPT (CPT@CND-SNP) for 24 hours. Fig. 6(e) illustrates the results of quantitative analysis of flow cytometry. Fig. 6(f) illustrates analysis of apoptosis-related biomarkers in U2OS cells by Western blotting. β-actin was used as an introduction control; CND concentration: 3.2 $\mu g\ mL^{-1}$, and CPT concentration: 10 $\mu g\ mL^{-1}$.

Fig. 7 illustrates the cancer cell elimination effect of chemically surface-modified CND composite nanoclusters that included various anti-cancer agents *in vitro.* Fig. 7(a) illustrates evaluation of cytotoxicity of chemically surface-modified CND composite nanoclusters that included various anti-cancer agents (curcumin, carmofur, cisplatin, and methotrexate) on U2OS cells; incubation time: 24 hours. Fig. 7(b) illustrates evaluation of cytotoxicity of a chemically surface-modified CND composite nanocluster as itself (CND-SNP) and PFOA on human normal fibroblasts (TIG-3). Fig. 7(c) illustrates evaluation of cytotoxicity of CPT, PFOA that included CPT, and a chemically surface-modified CND composite nanocluster that included CPT (CPT@CND-SNP) on U2OS cells; incubation time: 24 hours.

Fig. 8 illustrates the cancer cell elimination effect of chemically surface-modified CND composite nanoclusters that included CPT *in vivo.* Fig. 8(a) illustrates changes over time observed in nude mice with HT-29 solid cancer to which CPT (2.78 mg $kg^{-1}$), a chemically surface-modified CND composite nanocluster that included CPT (CPT@CND-SNP, 3.56 mg $kg^{-1}$), a chemically surface-modified CND composite nanocluster that included no CPT (CND-SNP), or a PBS buffer was intraperitoneally administered. Fig. 8(b) illustrates changes over time in tumor volume ratio after individual treatment. Fig. 8(c) illustrates changes over time in mouse body weight after individual treatment. Fig. 8(d) illustrates the tumor size at the end point after individual treatment.

Fig. 9 illustrates the average body weight of mice to which a chemically surface-modified CND composite nanocluster (CND-SNP, 3.56 mg $kg^{-1}$) or a PBS buffer was orally administered.

Fig. 10 illustrates a photograph of aqueous solutions of CND composite nanoclusters that contained carboxylated CND (CND-COOH) or aminated CND (CND-$NH_2$) to which an alkyl chain with a different length (C8: octyl, C12: dodecyl, C18: oleyl) was introduced on the surface; and also illustrates measurement values by dynamic light scattering. Aminated CND can be produced, for example, in accordance with the following documents: A. Krueger, Chem. Eur. J. 2008, 14, 1382-1390; Y. Liu, Chem. Mater. 2004, 16, 3924-3930; A. Krueger, Langmuir 2008, 24, 4200-4204; Denisov S.A., IOP Conf. Series: Materials Science and Engineering 16 (2010) 012005; and A. Krueger, Advanced Functional Materials, 22(5), 890-906.

Description of Embodiments

In the present specification, the "carbon nanomaterial" includes carbon nanodiamonds and carbon nanodots. The carbon nanomaterial according to the present invention is modified with a functional group represented by the following formula (I):

$$-Y^1-R \qquad (I)$$

wherein $Y^1$ represents a divalent linking group, and R represents an organic group. The carbon nanomaterial modified with this functional group may be referred to as "modified carbon nanomaterial."

The carbon nanomaterial according to the present invention has one, or two or more functional groups represented by formula (I). The functional group represented by formula (I) accounts for about 0.0001 to 30 mass%, preferably about 0.001 to 20 mass%, preferably about 0.01 to 15 mass%, and preferably about 0.05 to 10 mass% of the modified carbon nanomaterial.

The divalent linking group represented by $Y^1$ includes -NH-, -O-, -CO-O-, -O-CO-, -CO-NH-, -NH-CO-, -NH-CO-O-, -O-CO-NH-, -O-CO-O-, and -NH-CO-NH-.

The organic group represented by R includes a fluoroorganic group, a perfluoroorganic group, a substituted or unsubstituted alkyl group, a substituted or unsubstituted cycloalkyl group, a substituted or unsubstituted aryl group, a substituted or unsubstituted aralkyl group, and a substituted or unsubstituted heterocyclic group. The organic group is preferably a fluoroorganic group or a perfluoroorganic group.

The fluoroorganic group or perfluoroorganic group represented by R includes a substituted or unsubstituted fluoroalkyl group, a substituted or unsubstituted fluorocycloalkyl group, a substituted or unsubstituted fluoroaryl group, a substituted or unsubstituted fluoroaralkyl group, a substituted or unsubstituted fluoroheterocyclic group, a substituted

or unsubstituted perfluoroalkyl group, a substituted or unsubstituted perfluorocycloalkyl group, a substituted or unsubstituted perfluoroaryl group, a substituted or unsubstituted perfluoroaralkyl group, and a substituted or unsubstituted perfluoroheterocyclic group.

The substituent of the substituted alkyl group, fluoroalkyl group, perfluoroalkyl group, cycloalkyl group, fluorocycloalkyl group, perfluorocycloalkyl group, aryl group, fluoroaryl group, perfluoroaryl group, aralkyl group, fluoroaralkyl group, perfluoroaralkyl group, heterocyclic group, fluoroheterocyclic group, and perfluoroheterocyclic group includes halogen atoms, $C_{1-4}$ alkoxy, aryloxy, aralkyloxy, $C_{1-4}$ alkyl, $C_{2-4}$ alkenyl, NO, $NO_2$, $NH_2$, CN, OH, SH, COOH, $CONH_2$, $NHCOCH_3$, monoalkylamino, dialkylamino, monoalkylaminocarbonyl, dialkylaminocarbonyl, alkoxycarbonyl, and aryloxycarbonyl. The number of substituents of each substituted functional group is 1 to 5, preferably 1 to 3, and more preferably 1 or 2. Preferable substituents include a fluorine atom, a chlorine atom, aryloxy, aralkyloxy, $C_{1-4}$ alkyl, $NH_2$, CN, OH, COOH, monoalkylamino, dialkylamino, and alkoxycarbonyl.

The substituted or unsubstituted alkyl group includes substituted alkyl groups and unsubstituted alkyl groups (simply "alkyl group" below). The alkyl group includes linear or branched $C_{1-24}$ alkyl groups, such as methyl, ethyl, n-propyl, isopropyl, n-butyl, isobutyl, tert-butyl, n-pentyl, isopentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, docosyl, tricosyl, and tetracosyl.

The fluoroalkyl group includes linear or branched $C_{1-24}$ fluoroalkyl in which some hydrogen atoms of alkyl are substituted with a fluorine atom; and the perfluoroalkyl group includes linear or branched $C_{1-24}$ perfluoroalkyl in which all of the hydrogen atoms of alkyl are substituted with a fluorine atom.

The linear or branched $C_{1-24}$ fluoroalkyl includes $C_pH_qF_r$ (p represents an integer of 1 to 24, q represents an integer of 1 to 2p, r represents an integer of 1 to 2p, and $q + r = 2p + 1$). The linear or branched $C_{1-24}$ perfluoroalkyl includes $C_sF_{2s+1}$ (s represents an integer of 1 to 24).

The substituted or unsubstituted cycloalkyl group includes substituted cycloalkyl groups and unsubstituted cycloalkyl groups (simply "cycloalkyl group" below). The cycloalkyl group includes cyclopropyl, cyclobutyl, cyclopentyl, cyclohexyl, cycloheptyl, and cyclooctyl.

The fluorocycloalkyl group includes groups formed by replacing some hydrogen atoms of cycloalkyl with a fluorine atom (e.g., monofluorocyclopentyl and difluorocyclopentyl). The perfluorocycloalkyl group includes groups formed by replacing all of the hydrogen atoms of cycloalkyl with a fluorine atom (e.g., perfluorocyclopentyl and perfluorocyclohexyl).

The substituted or unsubstituted aryl group includes substituted aryl groups and unsubstituted aryl groups (simply "aryl group" below). The aryl group includes phenyl, naphthyl, fluorenyl, anthryl, biphenylyl, tetrahydronaphthyl, chromanyl, 2,3-dihydro-1,4-dioxanaphthalenyl, indanyl, and phenanthryl.

The fluoroaryl group includes groups formed by replacing some hydrogen atoms of aryl with a fluorine atom (e.g., fluorophenyl, difluorophenyl, and trifluorophenyl). The perfluoroaryl group includes groups formed by replacing all of the hydrogen atoms of aryl with a fluorine atom (e.g., perfluorophenyl, perfluorobiphenyl, and perfluoronaphthyl).

The substituted or unsubstituted aralkyl group include substituted aralkyl groups and unsubstituted aralkyl groups (simply, "aralkyl group" below). The aralkyl group includes benzyl, naphthylmethyl, fluorenylmethyl, anthrylmethyl, biphenylylmethyl, tetrahydronaphthylmethyl, chromanylmethyl, 2,3-dihydro-1,4-dioxanaphthalenylmethyl, indanylmethyl, phenanthrylmethyl, phenethyl, naphthylethyl, fluorenylethyl, anthrylethyl, biphenylylethyl, tetrahydronaphthylethyl, chromanylethyl, 2,3-dihydro-1,4-dioxanaphthalenylethyl, indanylethyl, and phenanthrylethyl.

The fluoroaralkyl group includes groups formed by replacing some hydrogen atoms of aralkyl with a fluorine atom (e.g., monofluorobenzyl, difluorobenzyl, trifluorobenzyl, monofluorophenethyl, difluorophenethyl, and trifluorophenethyl). The perfluoroaralkyl group includes groups formed by replacing all of the hydrogen atoms of aralkyl with a fluorine atom (e.g., perfluorobenzyl and perfluorophenethyl).

The substituted or unsubstituted heterocyclic group includes substituted heterocyclic groups and unsubstituted heterocyclic groups (simply "heterocyclic group" below). The heterocyclic group includes furyl, thienyl, pyrrolyl, imidazolyl, pyrazolyl, oxazolyl, thiazolyl, isooxazolyl, isothiazolyl, pyridyl, pyrazinyl, pyrimidinyl, pyridazinyl, indolyl, quinolyl, isoquinolyl, benzo[b]thienyl, and benzimidazolyl.

The fluoroheterocyclic group includes groups formed by replacing some hydrogen atoms of a heterocyclic group with a fluorine atom (e.g., monofluorofuryl, monofluoropyridyl, and monofluoroindolyl). The perfluoroheterocyclic group includes groups formed by replacing all of the hydrogen atoms of a heterocyclic group with a fluorine atom (e.g., perfluorofuryl, perfluorothienyl, perfluoropyrrolyl, and perfluoroindolyl).

The "halogen atom" refers to fluorine, chlorine, bromine, and iodine. The halogen atom is preferably fluorine, chlorine, or bromine, more preferably fluorine or chlorine, and particularly preferably fluorine.

The $C_{1-4}$ alkoxy includes methoxy, ethoxy, n-propoxy, isopropoxy, n-butoxy, isobutoxy, sec-butoxy, and tert-butoxy.

The aryloxy includes phenyloxy, naphthyloxy, fluorenyloxy, anthryloxy, biphenylyloxy, tetrahydronaphthyloxy, chromanyloxy, 2,3-dihydro-1,4-dioxanaphthalenyloxy, indanyloxy, and phenanthryloxy.

The aralkyloxy includes benzyloxy, naphthylmethyloxy, fluorenylmethyloxy, anthrylmethyloxy, biphenylylmethyloxy, tetrahydronaphthylmethyloxy, chromanylmethyloxy, 2,3-dihydro-1,4-dioxanaphthalenylmethyloxy, indanylmethyloxy, phenanthrylmethyloxy, phenethyloxy, naphthylethyloxy, fluorenylethyloxy, anthrylethyloxy, biphenylylethyloxy, tetrahydronaphthyl ethyloxy, chromanylethyloxy, 2,3-dihydro-1,4-dioxanaphthalenylethyloxy, indanylethyloxy, and phenanthryl ethyloxy.

The $C_{1-4}$ alkyl includes methyl, ethyl, n-propyl, isopropyl, n-butyl, isobutyl, and tert-butyl.

The $C_{2-4}$ alkenyl includes vinyl, allyl, and 1-butenyl.

The monoalkylamino includes methylamino, ethylamino, n-propylamino, isopropylamino, n-butylamino, isobutylamino, tert-butylamino, n-pentylamino, isopentylamino, and hexylamino.

The dialkylamino includes dimethylamino, diethylamino, di-n-propylamino, diisopropylamino, di-n-butylamino, diisobutylamino, di-tert-butylamino, di-n-pentylamino, diisopentylamino, and dihexylamino.

The monoalkylaminocarbonyl includes methylaminocarbonyl, ethylaminocarbonyl, n-propyl amino carbonyl, isopropylaminocarbonyl, n-butylaminocarbonyl, isobutylaminocarbonyl, tert-butylaminocarbonyl, n-pentylaminocarbonyl, isopentylaminocarbonyl, and hexylaminocarbonyl.

The dialkylaminocarbonyl includes dimethylaminocarbonyl, diethylaminocarbonyl, di-n-propylaminocarbonyl, diisopropylaminocarbonyl, di-n-butylaminocarbonyl, diisobutylaminocarbonyl, di-tert-butylaminocarbonyl, di-n-pentylaminocarbonyl, diisopentylaminocarbonyl, and dihexylaminocarbonyl.

The alkoxycarbonyl includes methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, isopentyloxycarbonyl, and hexyloxycarbonyl.

The aryloxycarbonyl includes phenyloxycarbonyl, naphthyloxycarbonyl, fluorenyloxycarbonyl, anthryloxycarbonyl, biphenylyloxycarbonyl, tetrahydronaphthyloxycarbonyl, chromanyloxycarbonyl, 2,3-dihydro-1,4-dioxanaphthalenyloxycarbonyl, indanyloxycarbonyl, and phenanthryloxycarbonyl.

Carbon nanomaterials having many groups such as OH, COOH, or $NH_2$ on the surface thereof are known ("CNM" below). The carbon nanomaterial modified with a functional group according to the present invention can be obtained using these materials in accordance with the following schemes (1) to (10).

Schemes

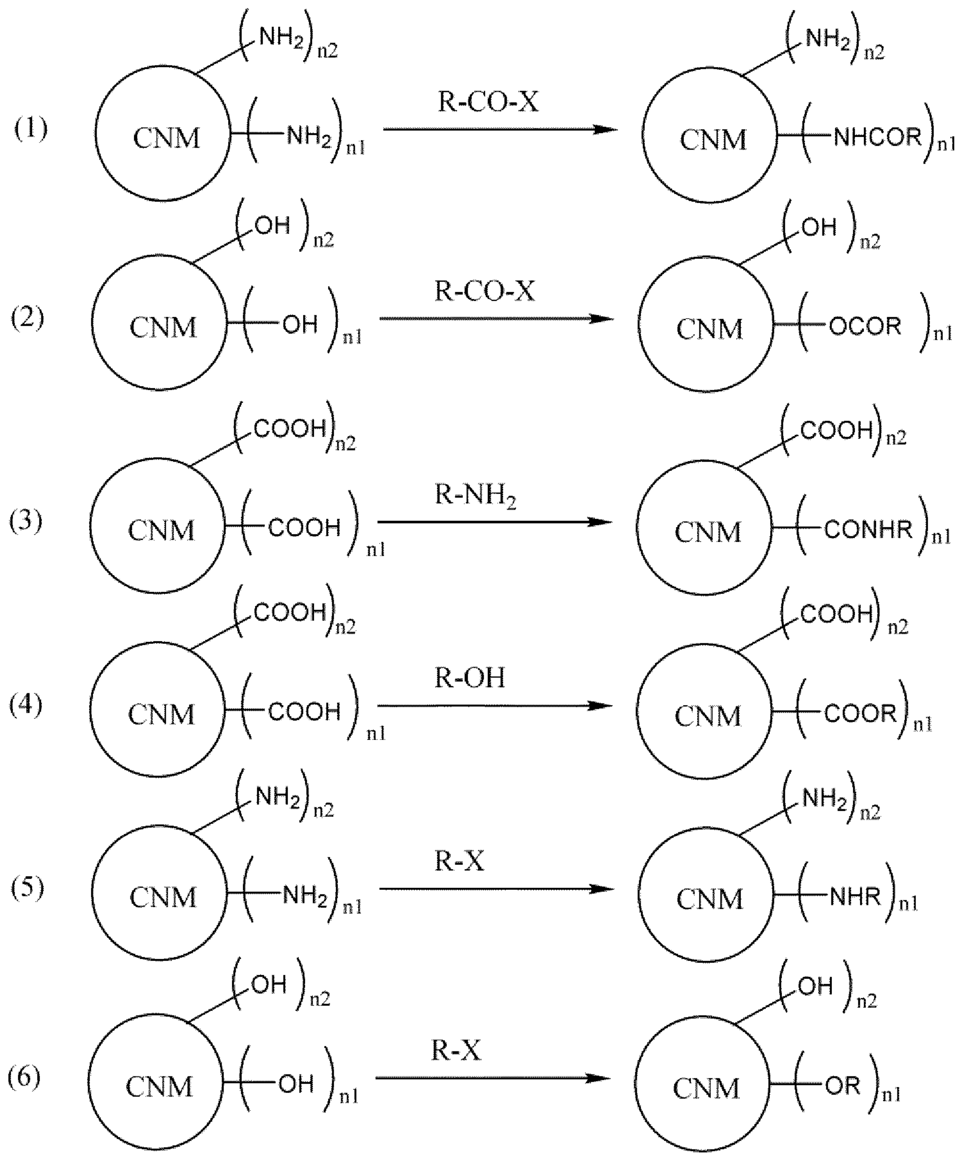


(1)

(2)

(3)

(4)

(5)

(6)

(7) (8) (9) (10)

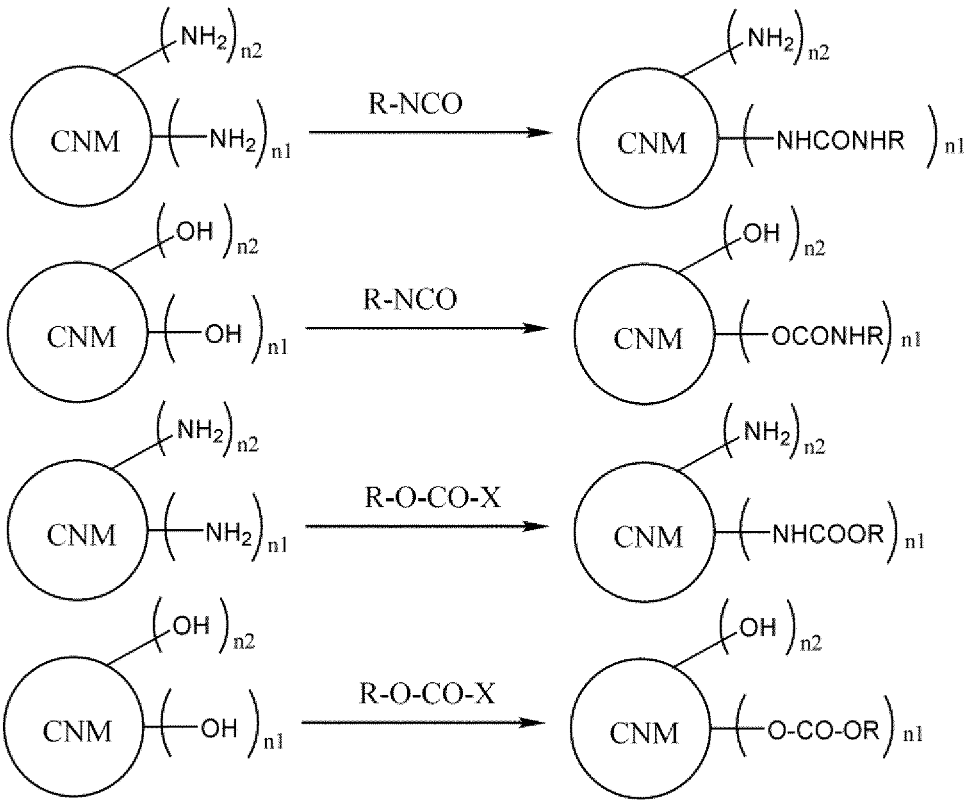


wherein X represents Cl, Br, or I; CNM represents a carbon nanomaterial; n1 represents an integer of 1 or more; n2 represents an integer of 0 or more; and R is defined as above.

The reactions in schemes (1) to (10) can be performed in accordance with an ordinary method. The target product can be obtained by reacting 1 g of a carbon nanomaterial having one or more surface groups (OH, $NH_2$, or COOH) with 1 mg to an excessive amount of a compound selected from R-COX, R-$NH_2$, R-OH, and R-X at a temperature of 0°C to a boiling temperature of a solvent for 1 to 24 hours. The solvent includes halogenated hydrocarbons, such as chloroform, methylene chloride, and 1,2-dichloroethane; aromatic hydrocarbons, such as toluene; tetrahydrofuran; diethyl ether; and diisopropyl ether.

The nanocluster according to the present invention can be prepared by suspending a carbon nanomaterial modified with a functional group in a suitable aqueous vehicle, such as water or a buffer, and exposing the suspension to ultrasound to cause the carbon nanomaterial to self-assemble. After exposure to ultrasound, self-assembled nanoclusters can be separated by removing the unreacted functional-group-modified carbon nanomaterial that is not in the form of a cluster, by using a purification operation such as centrifugation.

The nanocluster according to the present invention can be formed into a composite with an active ingredient by dispersing the nanocluster in a suitable solvent containing the active ingredient. The active ingredient is present on the surface of the nanocluster, or inside the nanocluster. The active ingredient includes physiologically active substances, labeling substances, essential oils, flavors, and organic dyes.

Labeling substances include fluoresceins, such as fluorescein, Oregon Green, eosine, and erythrosine; rhodamines, such as tetramethyl rhodamine derivatives, Texas Red derivatives, rhodamine B base, Lissamine rhodamine B, and rhodamine 6G; coumarins; dansyl-type (dimethylamino naphthalene sulfonic acid-type) fluorescent dyes; NBD-type dyes; pyrene; phycobiliproteins, such as R-phycoerythrin, phlophycocyanin, and allophycocyanin; BODIPY derivatives; Cy (registered trademark) dyes, such as Cy3, Cy3.5, Cy5, and Cy5.5; and Alexa Fluor (registered trademark), such as Alexa Fluor 350, 405, 430, 488, 532, 546, 555, 568, 594, 633, 647, 680, 700, and 750. These labeling substances may be used singly, or in a combination of two or more.

Essential oils include sweet orange, bitter orange, petitgrain, lemon, grapefruit, lime, bergamot, mandarin, neroli, peppermint, spearmint, lavender, chamomile, rosemary, eucalyptus, sage, basil, rose, geranium, jasmine, ylang ylang, anise, fennel, star anise, clove, cinnamon, ginger, nutmeg, cardamom, hop, Japan cedar, cypress, vetiver, patchouli, and labdanum. These essential oils may be used singly, or in a combination of two or more.

The components of flavors include monoterpenes, such as 1-menthol, α-pinene, β-pinene, myrcene, camphene, and limonene; sesquiterpenes, such as valencene, cedrene, caryophyllene, and longifolene; terpene ketones, such as 1,3,5-undecatrien, butanol, pentanol, isoamyl alcohol, hexanol, prenol, (Z)-3-hexen-1-ol, 2,6-nonadienol, linalool, geraniol, citronellol, tetrahydromyrcenol, farnesol, nerolidol, cedrol, benzyl alcohol, phenyl ethyl alcohol, furfuryl alcohol, acetaldehyde, isovaleraldehyde, hexanal, octanal, nonanal, decanal, (E)-2-hexenal, 2,4-octadienal, citronellal, citral, benzaldehyde, cinnamaldehyde, vanillin, ethylvanillin, furfural, heliotropine, 2-heptanone, 2-undecanone, 1-octen-3-one, acetoin, diacetyl, 2,3-pentanedione, maltol, ethyl maltol, 2,5-dimethyl- 4-hydroxy-3(2H)-furanone, hydroxyketone, carvone, menton, and nootkatone; and α-ionone, β-ionone, β-damascenone, raspberry ketone, rose oxide, linalool oxide, menthofuran, theaspirane, methyl chavicol, anethole, ethyl acetate, isoamyl acetate, linalyl acetate, geranyl acetate, lavanjuril acetate, ethyl butyrate, ethyl caproate, benzyl acetate, methyl salicylate, γ-decalactone, γ-dodecalactone, δ-decalactone, δ-dodecalactone, 7-decene-4-olide, 2-decene-5-olide, butyric acid, 4-methyl-3-pentenoic acid, octanoic acid, stearic acid, oleic acid, linoleic acid, linolenic acid, indole, skatole, pyridine, alkylated pyrazine, methyl anthranilate, methanethiol, furfuryl mercaptan, dimethylsulfide, dimethyldisulfide, difurfuryl disulfide, and allyl isothiocyanate. These components may be used in a combination of two or more.

Natural dyes include annatto dyes, cochineal dyes, monascus dyes, β-carotene, hibiscus dyes, sulfur yellow, cacao dyes, riboflavin, chlorophyll, caramel, annotto, carmine, laccainic acid, brazilin, crocin, shikonin, shisonin, and rutin.

When being administered or applied internally to or on the skin of a mammal (e.g., humans, mice, rats, hamsters, horses, cow, swine, goats, sheep, rabbits, dogs, and cats), the nanocluster according to the present invention, together with an active ingredient, in the form of a composite releases the active ingredient in an extended manner. Thus, the nanocluster according to the present invention in the form of a composite with an active ingredient is useful as a pharmaceutical product or pharmaceutical composition, a cosmetic, or an oral composition. Cosmetics include foundation, face powder, lotions, milky lotions, lip sticks, skin toners, beauty essences, massage cream, moisturizing cream, face pack, facial cleansers, shampoos, hair conditioners, hair growth stimulants, and body powder. Oral compositions include mouthwash, mouth rinse, toothpaste, dental powder, chewing gum, candies, gummy candies, *Ramune* candies, and *Ramune* soda. The dosage form of pharmaceutical products includes tablets, capsules, lozenges, pills, chewable tablets, injectable drugs, suppositories, syrups, ointments, and plasters. The nanocluster according to the present invention is also useful as a carrier for an active ingredient because the nanocluster releases the active ingredient in an extended manner.

Physiologically active substances include drugs, nucleic acids, and proteins. Drugs include antitumor agents, antihypertensives, antihypotensives, antipsychotics, analgesics, antidepressants, antimanics, anxiolytics, tranquilizers, hypnotics, antiepileptics, opioid agonists, asthma therapeutic agents, anesthetics, antiarrhythmic agents, arthritis therapeutic agents, antispasmodics, ACE inhibitors, decongestants, antibiotics, antianginal agents, diuretics, anti-Parkinson's disease agents, bronchodilators, antidiuretics, diuretics, antihyperlipidemics, immunosuppressants, immunomodulators, antiemetics, anti-infective agents, antineoplastics, antimycotic agents, antivirotics, antidiabetic agents, antiallergic agents, antipyretics, antigout agents, antihistamine agents, antipruritic agents, bone modifiers, cardiovascular agents, cholesterol lowering agents, antimalarial agents, antitussives, expectorants, mucolytic agents, nasal congestion medicines, dopamine agonists, gastrointestinal drugs, muscle relaxants, neuromuscular blockers, parasympathetic agonists, prostaglandins, stimulants, anorectic agents, thyroid agents or anti-thyroid agents, hormones, antimigraine agents, anti-obesity agents, and anti-inflammatory agents. A preferable drug is an antitumor agent. Antitumor agents include hormone therapy agents (e.g., fosfestrol, diethylstilbestrol, chlorotrianserine, medroxyprogesterone acetate, megestrol acetate, chlormadinone acetate, cyproterone acetate, danazol, allylestrenol, gestrinone, mepartricin, raloxifene, ormeloxifene, levormeloxifene, anti-estrogens (e.g., tamoxifen citrate, and toremifene citrate), pill preparations, mepitiostane, testololactone, aminoglutethimide, LH-RH agonists (e.g., goserelin acetate, buserelin, and leuprorelin), droloxifene, epitiostanol, ethinyl estradiol sulfonate, aromatase inhibitors (e.g., fadrozole hydrochloride, anastrozole, letrozole, exemestane, vorozole, and formestane), anti-androgens (e.g., flutamide, bicalutamide, and nilutamide), 5α-reductase inhibitors (e.g., finasteride, and epristeride), adrenal cortex hormone-based agents (e.g., dexamethasone, prednisolone, betamethasone, and triamcinolone), androgen synthesis inhibitors (e.g., abiraterone), retinoid, and agents for delaying the metabolism of retinoid (e.g., liarozole); in particular, LH-RH agonists (e.g., goserelin acetate, buserelin, and leuprorelin)), alkylating agents (e.g., nitrogen mustard, nitrogen mustard N-oxide hydrochloride, chlorambucil, cyclophosphamide, ifosfamide, thiotepa, carboquone, improsulfan tosilate, busulfan, nimustine hydrochloride, mitobronitol, melphalan, dacarbazine, ranimustine, estramustine phosphate sodium, triethylene melamine, carmustine, lomustine, streptozocin, pipobroman, etoglucid, carboplatin, cisplatin, miboplatin, nedaplatin, oxaliplatin, altretamine, ambamustin, dibrospidium hydrochloride, fotemustine, prednimustine, pumitepa, ribomustin, temozolomide, treosulfan, trofosfamide, zinostatin stimalamer, carboquone, adozelesin, cystemustine, and bizelesin), antimetabolites (e.g., mercaptopurine, 6-mercaptopurine riboside, thioinosine, methotrexate, enocitabine, cytarabine, cytarabine ocfosphate, ancitabine hydrochloride, 5-FU-based medicinal agents (e.g., fluorouracil, tegafur, UFT, doxifluridine, carmofur, galocitabine, and emitefur), aminopterin, calcium leucovorin, tabloid, butocin, calcium folinate, calcium levofolinate, cladribine, emitefur, fludarabine, gemcitabine, hydroxycarbamide, pentostatin, piritrexim, idoxuridine, mitoguazone, tiazofurin, ambamustin), anticancer antibiotics (e.g., actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarkomycin, carzinophilin, mitotane,

zorubicin hydrochloride, mitoxantrone hydrochloride, idarubicin hydrochloride), plant-derived anticancer agents (e.g., etoposide, etoposide phosphoate, vinblastine sulfate, vincristine sulfate, vindesine sulfate, teniposide, paclitaxel, docetaxel, vinorelbine, camptothecin, and irinotecan hydrochloride), immunotherapeutic drugs (BRM) (e.g., picibanil, krestin, schizophyllan, lentinan, ubenimex, interferon, interleukin, macrophage colony-stimulating factors, granulocyte colony-stimulating factors, erithropoietin, lymphotoxin, BCG vaccines, corynebacterium parvum, levamisole, polysaccharide K, and procodazole), and medicinal agents for inhibiting action of cell growth factors and their receptors (e.g., antibody drugs such as trastuzumab (Herceptin (trademark), anti-HER2 antibody), ZD1839 (IRESSA), and Gleevec). The type of cancer targeted by the antitumor agents include colon and rectal cancer, liver cancer, kidney cancer, head and neck cancer, esophagus cancer, stomach cancer, biliary tract cancer, gallbladder and bile duct cancer, pancreas cancer, lung cancer, breast cancer, ovarian cancer, cervical cancer, endometrial cancer, bladder cancer, prostate cancer, testicular tumor, bone and soft tissue sarcoma, leukemia, malignant lymphoma, multiple myeloma, skin cancer, and brain tumor. The type of cancer targeted by the antitumor agents is preferably colon and rectal cancer, stomach cancer, head and neck cancer, lung cancer, breast cancer, pancreas cancer, biliary tract cancer, and liver cancer.

The nucleic acid is not particularly limited; and may be DNA, RNA, a chimeric nucleic acid of DNA and RNA, a hybrid of DNA/RNA, or the like. The nucleic acid for use may be in a single- to three-stranded form, and is preferably single-stranded or double-stranded. The nucleic acid may be another oligomer that has another type of a nucleotide framework including a purine base or pyrimidine base and an N-glycoside or that has a non-nucleotide framework (e.g., commercially available peptide nucleic acids (PNA)); or another oligomer having a unique bond (this oligomer contains nucleotides that have a conformation that allows for a base pair as found in DNA or RNA, or base adhesion). The nucleic acid may be those having a known modification added, such as nucleic acids with a labeling known in the art, capped nucleic acids, methylated nucleic acids, nucleic acids obtained by replacing at least one natural nucleotide with an analog, nucleic acids with intermolecular nucleotide modification, nucleic acids with uncharged bonds (e.g., methyl phosphonate, phosphotriester, phosphoramidate, and carbamate), nucleic acids with charged bonds or sulfur-containing bonds (e.g., phosphorothioate, and phosphorodithioate), nucleic acids with a side chain group, such as a protein (nucleases, nuclease inhibitors, toxins, antibodies, and signal peptides) or sugar (e.g., monosaccharide), nucleic acids with an intercalate compound (e.g., acridine and psoralen), nucleic acids with a chelate compound (e.g., metals, radioactive metals, boron, and oxidative metals), nucleic acids with an alkylating agent, or nucleic acids with a modified bond (e.g., $\alpha$-anomeric nucleic acids). Preferable nucleic acids include RNAs, such as siRNA.

siRNA refers to a double-stranded oligo RNA composed of a nucleotide sequence homologous to the nucleotide sequence of mRNA of a target gene or its initial transcript, or a partial sequence of the nucleotide sequence (preferably within the coding region; if the nucleotide sequence is of an initial transcript, the sequence includes intronic regions); and a complementary strand of the nucleotide sequence. Although the length of the portion homologous to the target nucleotide sequence contained in siRNA is typically about 18 bases or more; and, for example, around about 20 bases (typically about 21 to 23 base length), the length can be any length that can cause RNA interference. Although the full length of siRNA is typically about 18 bases or more; for example, around about 20 bases (typically about 21 to 23 base length), the full length of siRNA can be any length that can cause RNA interference.

The relation between the target nucleotide sequence and the sequence homologous to the target nucleotide sequence contained in siRNA may be a 100% match, or there may be a base mutation (their identity can be within the range of at least 70%, preferably 80%, more preferably 90%, and most preferably 95% or more).

siRNA may have additional bases at 5' or 3' end that are composed of 5 bases or less, preferably 2 bases, and that do not form a base pair. Although the additional bases may be DNA or RNA, the use of DNA can increase the stability of siRNA. Examples of sequences of such additional bases include, but are not limited to, sequences such as ug-3', uu-3', tg-3', tt-3', ggg-3', guuu-3', gttt-3', ttttt-3', and uuuuu-3'.

The siRNA may be for any target gene. Preferable siRNA includes those whose increased expression targets a gene involved in the onset and/or exacerbation of a target disease; more specifically, preferable siRNA includes those formed such that the antisense nucleic acid of the gene targets genes that are in the clinical or preclinical phase or newly found genes.

The siRNA for use may be one type alone, or a combination of two or more types.

Proteins include enzymes, receptors, antibodies, antigens, and cytokines, such as interferons and interleukins.

The nanocluster according to the present invention has an average particle size of about 1 to 1000 nm, preferably about 3 to 800 nm, more preferably about 5 to 500 nm, still more preferably about 10 to 300 nm, and particularly preferably about 30 to 250 nm.

The nanocluster has a zeta potential of preferably about 5 to 30 mV, and more preferably about 10 to 25 mV.

The nanocluster according to the present invention in the form of a composite with an active ingredient contains the active ingredient in an amount of about 5 to 50 parts by mass, and preferably about 10 to 20 parts by mass, per 100 parts by mass of the nanocluster.

Examples

Below, the present invention is described in more detail with reference to Examples. However, the present invention is not limited to the Examples.

Example 1

Experimental Method

Synthesis of Chemically Surface-Modified CND Composite Nanocluster (CND-SNP Cluster)

A chemically surface-modified CND composite nanocluster was synthesized in accordance with the following method (Fig. 2(a)).

An aqueous solution of CND-ori that had an amino group on the surface thereof (1 mL, CND concentration: 3.2 mg/mL; Daicel, Tokyo, Japan), perfluorooctanoic acid (PFOA) (10 mg; Sigma-Aldrich, St. Louis, MO, USA), and water-soluble carbodiimide (10 mg; WSC, Wako, Osaka, Japan) were subjected to sonication for 5 minutes with a bath-type ultrasound device (USD-2R; power output, 80W; oscillation frequency, 40 kHz; AS ONE), thereby dissolving these substances in a 2-(N-morpholino)ethanesulfonic acid (MES) buffer (9 mL) (pH 6.0, 100 mM). After intense stirring at room temperature for 5 minutes, the mixture was washed with Milli-Q water three times using centrifugation to remove unreacted compounds. Pellets generated by centrifugation were subjected to sonication for 10 minutes with a pulse-type ultrasound device (VCX-600; Sonics, Danbury, CT, USA), thereby dispersing the pellets in distilled water (10 mL). Centrifugation (VCX-600; Sonics, Danbury, CT, USA) (1,000 rpm, 10 minutes, 20°C) was performed to remove impurities formed by the pulse-type ultrasound device. The supernatant having a chemically surface-modified CND composite nanocluster (CND-SNP cluster) dispersed therein was used in the experiments below.

The method for preparing a chemically surface-modified CND composite nanocluster in which BODIPY or CPT is included (BODIPY@CND-SNP and CPT@CND-SNP) is as follows. Distilled water (10 mL), and BODIPY (1 mg, Sigma-Aldrich) or camptothecin (CPT) (10 mg, Wako) were added to CND pellets chemically modified with PFOA as described above (CND-SNP cluster), and the mixture was subjected to sonication for 10 minutes with a bath-type ultrasound device. The prepared mixture was stored at 4°C, and then used in biological experiments. Fig. 1(d) illustrates a chemically surface-modified CND composite nanocluster that includes CPT (CPT@CND-SNP). Fig. 4(a) illustrates the production steps for a chemically surface-modified CND composite nanocluster that includes BODIPY (BODIPY@CND-SNP).

Characterization of Synthesis of Chemically Surface-Modified CND Composite Nanocluster

The nanostructure of the CND before having been subjected to chemical surface modification (CND-ori) and the chemically surface-modified CND composite nanocluster (CND-SNP cluster) was analyzed with a high-resolution transmission electron microscope (TEM) (EM-002B; Topcon, Tokyo, Japan) (120 kV) (Fig. 2(d) and Fig. 3(b)). The hydrodynamic size of the chemically surface-modified CND composite nanocluster was investigated by dynamic light scattering (DLS) (Photal FPAR-1000; Otsuka Electronics, Osaka, Japan) (Fig. 2(c)). The optical properties and concentration of the samples were determined with a UV-VIS-NIR spectrometer (V-730BIO; Jasco, Tokyo, Japan) (Fig. 2(b), Fig. 4(b), and Fig. 5(a)). The chemically surface-modified CND composite nanocluster (CND-SNP cluster) and unmodified CND powder (CND-ori) (2 mg each) were burned in an oxygen gas atmosphere (10°C $min^{-1}$) using a thermogravimetric analyzer (TGA Q 500; TA Instrument, New Castle, DE, USA), and the amount of PFOA used in chemical surface modification was analyzed (Fig. 3(a)). The fluorescence properties of the chemically surface-modified CND composite nanocluster was observed with a fluorescence spectrometer (FP-6500; Jasco) before and after BODIPY was included (Fig. 4(b) and Fig. 5(a)).

Amino groups in the chemically surface-modified CND composite nanocluster and unmodified CND were quantified with a Kaiser test kit (60017-1EA, Sigma-Aldrich) in the manner described below. The chemically surface-modified CND composite nanocluster or unmodified CND powder (0.64 mg) was added to distilled water (200 μL). Subsequently, solution 1 (200 μL, Phenol, 80% in ethanol) and solution 2 (200 μL, KCN in $H_2O$/ pyridine) were added to the aqueous solution, and the mixture was subjected to sonication with a bath-type ultrasound device until powder derived from the CND was well-dispersed. The mixture was then heated with a heat block (EB-603; AS ONE) at 120°C for 10 minutes; and solution 3 (200 μL, Ninhydrin, 6% in ethanol) was added thereto, followed by further heating for 10 minutes. The obtained solution was cooled at room temperature for 30 minutes, and 60% ethanol was added thereto to dilute the solution such that the total amount became 3 mL. After centrifugation (15,000 rpm, 5 minutes), the UV-visible spectrum of the supernatant was analyzed. The absorbance at 570 nm was calculated as free amino groups on the CND surface using the following formula (Fig. 3(c)).

$$NH_2\ loading\ (\mu mol/g) = \frac{[Abs_{sample} - Abs_{blank}] \times dilution\ (mL) \times 10^6}{Extinction\ coefficient\ sample\ weight\ (mg)}$$

The dilution amount was 3 mL, and the extinction coefficient was 15000 $m^{-1}$ $cm^{-1}$. A blank sample was precisely prepared using distilled water in the same manner. The result was calculated with the amount of amino groups of unmodified CND taken as 100%.

The zeta potential of the chemically surface-modified CND composite nanocluster (CND-SNP) was measured by Kyowa Interface Science Co., Ltd. (Saitama, Japan) with a zeta potential analyzer (ZEECOM ZC-3000; Microtec Co., Ltd., Chiba, Japan) (Fig. 3(d)).

The interfacial tension of the chemically surface-modified CND composite nanocluster (CND-SNP) was measured by Mitsui Chemical Analysis & Consulting Service, Inc. (Tokyo, Japan) with a fully automatic surface tensiometer (CBVP-Z; Kyowa Interface Science Co., Ltd.) (Fig. 3(e)).

Cell Culture and Evaluation of Cytotoxicity

Human colon adenocarcinoma (HT-29), human osteosarcoma cells (U2OS), human cervical cancer (HeLa), and human normal fibroblasts (TIG3) were purchased from JCRB Cell Bank or DS Pharma Biomedical (Tokyo, Japan). These cells were cultured in a DMEM medium (Gibco BRL, Grand Island NY, USA) that contained fetal bovine serum (10%), L-glutamine (2 mM), sodium pyruvate (1 mM), gentamycin, penicillin-streptomycin (100 IU $mL^{-1}$), and Hank's balanced salt solution (Life Technologies, MD, USA) at 37°C in 5% carbon dioxide. Morphological observation on cells was recorded with a phase-contrast microscope (Nikon Eclipse TE300, Japan) (Fig. 5(b)). Evaluation of cytotoxicity was performed using crystal violet staining (Wako) and a Cell Counting Kit-8 (CCK-8; Dojindo Laboratories, Kumamoto, Japan). In CCK-8 assay, the cells were seeded in a 96-well plate (7000 cells $well^{-1}$), and incubated overnight for adhesion. After incubation, the cells were exposed to a drug or various nanomaterials. After being washed with a fresh medium, the cells were incubated with a CCK-8 solution in accordance with the instruction manual of the CCK-8 assay kit, and the absorbance at 450 nm was analyzed with a microplate reader (Infinite M200 Pro; Tecan, Switzerland). In the crystal violet assay, the cells were seeded in a 12-well plate ($1.5\times10^5$ cells $well^{-1}$), and then incubated overnight. After being washed with the PBS buffer used for cooling the cells, the cells were immobilized for 10 minutes with a methanol/acetone (v/v, 1:1) mixture that had been cooled beforehand. The immobilized cells were stained with a 0.1% crystal violet solution overnight. All of the experiments concerning cytotoxicity evaluation were performed at least 3 times (Fig. 1(e), Fig. 6(b), Fig. 6(c), and Fig. 7(a) to Fig. 7(c)).

Fluorescence Microscope Live Imaging

The cells were seeded in a glass-bottom dish for imaging, and incubated overnight for adhesion. Subsequently, the chemically surface-modified CND composite nanocluster including BODIPY was added to the cells, and incubated at 37°C or 4°C for 2 hours. After incubation, the cell nuclei were stained with Hoechst 33342 (1 $\mu$g $mL^{-1}$, Thermo Scientific, Waltham, MA, USA) for 10 minutes. Three minutes afterward, the cells were washed with a PBS buffer; and a phenol red-free RPMI 1640 medium (Thermo Scientific) was added thereto, followed by performing cell live imaging. To investigate the distribution of the chemically surface-modified CND composite nanocluster including BODIPY in cells, simultaneous staining was performed with LysoTracker Red (Thermo Scientific) in accordance with the manual in the manner below. First, the cells were treated with the chemically surface-modified CND composite nanocluster containing BODIPY for 4 hours, and then incubated with a LysoTracker reagent (100 nM) at 37°C for 2 hours. The cells were washed with a fresh PBS buffer 3 times, and incubated with Hoechst 33342 (1 $\mu$g $mL^{-1}$) for 10 minutes. The stained cells in a phenol red-free medium were observed with a fluorescence microscope (IX73; Olympus) equipped with a mirror unit (IX3-FGFPXL, Olympus) and an EM-CCD camera (DP80; Olympus) (Fig. 4(c) and Fig. 4(e)).

Western Blotting

The cells were harvested with a RIPA buffer (Thermo Scientific) to which a protease inhibitor cocktail (Roche Applied Science, Mannheim, Germany) was added. The concentration of each protein was determined using a Pierce BCA Protein Assay Kit (Thermo Scientific). Western blotting was performed in accordance with a published document (Non-patent Literature: Yu et al., Cell Death and Disease (2017) 8, e2755; doi:10.1038/cddis.2017.33), using the following antibodies: rabbit anti-LAMPI (ab24170; Abcam, Cambridge, MA, USA), anti-Bcl-xL (2762; Cell Signaling, MA, USA), anti-Bcl-2 (2876; Cell Signaling), anti-caspase-8(06-775; Millipore, Billerica, MA, USA), anti-caspase-3 (H-277; Santa Cruz, CA, USA), anti-PARP-1 (H-250; Santa Cruz), mouse anti-$\beta$-Tubulin (T5293; Sigma-Aldrich), anti-p53 (DO-1; Santa Cruz), and anti-$\beta$-actin (AC-15; Abcam) (Fig. 4(d) and Fig. 6(f)).

Flow Cytometry

Flow cytometry was performed by double-staining using Annexin-V and 7-AAD, which are capable of detecting apoptosis. The cells were seeded in a 6-well plate to give a density of $3\times10^5$ cells $well^{-1}$, and incubated overnight for adhesion. Twenty-four hours afterward, the cells were harvested; and an apoptosis assay was performed in accordance with a manual, and analyzed using a cocktail solution containing a Guava Nexin Reagent (Millipore), Annexin V-PE, and 7-AAD, with a Guava PCA flow cytometer (Millipore). The proportion of apoptotic cells was calculated using FlowJo software (Version 7.6; FlowJo LLC, Oregon, USA) (Fig. 6(d) and Fig. 6(e)).

*In Vivo* Anticancer Experiment and Toxicity Evaluation

Five-week-old female nude mice (n = 5; average weight = 18 g; C57BL/6NCrSlc) were purchased from Japan SLC, Inc. (Shizuoka, Japan), and allowed to become acclimated in a specific pathogen-free (SPF) environment for 1 week. All of the animal experiments were performed with the approval of the animal research committees of The National Institute of Advanced Industrial Science and Technology and Japan SLC, Inc. A mixture solution of a HT-29 cell dispersion ($1\times10^6$ cells) (100 μL) and a culture medium/Matrigel (Corning, NY, USA) (v/v, 1:1) was subcutaneously administered to the abdomen of the mice to prepare cancer model mice. Eight days afterward, 200 μL of the chemically surface-modified CND composite nanocluster (3.56 mg $kg^{-1}$), CPT (2.78 mg $kg^{-1}$), the chemically surface-modified CND composite nanocluster including CPT (CPT, 2.78 mg kg-1; CND-SNP, 3.56 mg kg-1), or a PBS buffer was intraperitoneally administered to the mice that had a solid cancer. The behavior and characteristics of the mice, solid cancer size, and changes in body weight of the mice were measured once every two days (Figs. 8(a) to 8(d)). The size of the solid cancer was calculated using the following formula.

$$V = L \times W^2/2$$

In the formula, V represents the volume of the solid cancer, L represents the length of the solid cancer, and W represents the width of the solid cancer.

The evaluation of *in vivo* toxicity of the chemically surface-modified CND composite nanocluster (CND-SNP) was performed in the following manner. The chemically surface-modified CND composite nanocluster (3.56 mg $kg^{-1}$) or a PBS buffer was orally administered to 5-week-old female nude mice (n = 5; average weight = 18 g, C57BL/6NCrSlc); and the body weight of the mice (Fig. 9) as well as the behavior and characteristics of the mice were carefully investigated for 28 days, once every two days. A mouse blood test was performed by administering 200 μL of the chemically surface-modified CND composite nanocluster (3.56 mg $kg^{-1}$) or a PBS buffer to 11-week-old female mice (n = 6; average weight = 21 g, BALB/cAJcl) through their tail vein, and collecting blood through their abdominal aorta 28 days later. The complete blood cell count (CBC) and biochemical test were analyzed by Japan SLC, Inc. and Oriental Yeast Co., Ltd. (Tokyo, Japan).

Table 1

The complete blood cell count (CBC) of mice administered with a chemically surface-modified CND composite nanocluster through their tail vein, and biochemical test

| Method | Entry | Unit | PBS (n = 5) | CND-SNP (n = 5) | p value |
|---|---|---|---|---|---|
| Complete Blood Count (CBC) | WBC | $\times 10^{2}$/µL | 90.67 ± 21.04 | 88.83 ± 13.53 | > 0.05 |
| | RBC | $\times 10^{4}$/µL | 977.67 ± 30.66 | 967.33 ± 20.09 | > 0.05 |
| | PLT | $\times 10^{4}$/µL | 70.32 ± 4.37 | 70.80 ± 1.67 | > 0.05 |
| Biochemical examination | CRP | µg/mL | 1.62 ± 0.12 | 1.62 ± 0.16 | > 0.05 |
| | TP | g/dL | 4.22 ± 0.24 | 4.12 ± 0.13 | > 0.05 |
| | ALB | g/dL | 2.77 ± 0.14 | 2.63 ± 0.14 | > 0.05 |
| | BUN | mg/dL | 15.24 ± 1.27 | 16.34 ± 0.61 | > 0.05 |
| | CRE | mg/dL | 0.13 ± 0.012 | 0.13 ± 0.009 | > 0.05 |
| | AST | IU/L | 45.20 ± 6.02 | 51.80 ± 4.02 | > 0.05 |
| | LDH | IU/L | 183.33 ± 28.08 | 221.33 ± 54.96 | > 0.05 |
| | AMY | IU/L | 1777.6 ± 206.49 | 1661.4 ± 127.96 | > 0.05 |
| | CK | IU/L | 218.00 ± 98.19 | 233.40 ± 54.45 | > 0.05 |

| | |
|---|---|
| WBC | White Blood Cell |
| RBC | Red Blood Cell |
| PLT | Platelets |
| CRP | C-Reactive Protein |
| TP | total protein |
| ALB | albumin |
| BUN | Blood Urea Nitrogen |
| CRE | creatinine |
| AST | aspartate aminotransferase |
| LDH | lactate dehydrogenase |
| AMY | amylase |
| CK | creatine kinase |

Statistical Analysis of Data

In these data, "±" indicates a standard deviation, and "n" indicates the number of samples used. Statistical analysis of these data was performed using the Student's t-test. The symbols "*," "**," and "***" respectively indicate p<0.05, p<0.005, and p<0.001.

## Claims

1. A self-assembled nanocluster comprising a carbon nanomaterial modified with a functional group represented by the following formula (I):

$$-Y^{1}-R \qquad (I)$$

wherein $Y^1$ represents a divalent linking group, and R represents an organic group.

2. The nanocluster according to claim 1, wherein the divalent linking group represented by $Y^1$ is one member selected from the group consisting of -NH-, -O-, -CO-O-, -O-CO-, -CO-NH-, -NH-CO-, -NH-CO-O-, -O-CO-NH-, -O-CO-O-, and -NH-CO-NH-.

3. The nanocluster according to claim 1 or 2, wherein the organic group represented by R is a fluoroorganic group, a perfluoroorganic group, a substituted or unsubstituted alkyl group, a substituted or unsubstituted cycloalkyl group, a substituted or unsubstituted aryl group, a substituted or unsubstituted aralkyl group, or a substituted or unsubstituted heterocyclic group.

4. The nanocluster according to any one of claims 1 to 3, wherein the carbon nanomaterial has at least one surface group selected from the group consisting of OH, COOH, and $NH_2$; and the functional group represented by formula (I) is incorporated in the carbon nanomaterial through the surface group.

5. The nanocluster according to any one of claims 1 to 4, which is, together with an active ingredient, in the form of a composite.

6. The nanocluster according to claim 5, wherein the active ingredient is a physiologically active substance, a labeling substance, a flavor, an essential oil, or an organic dye.

7. The nanocluster according to any one of claims 1 to 6, wherein the carbon nanomaterial is a carbon nanodiamond or a carbon nanodot.

**8.** A carrier for an active ingredient, the carrier comprising the nanocluster of any one of claims 1 to 7.

**9.** A carbon nanomaterial modified with a functional group represented by the following formula (I):

$$-Y^1-R \qquad (I)$$

wherein $Y^1$ represents a divalent linking group, and R represents a fluoroorganic group or a perfluoroorganic group.

**10.** The carbon nanomaterial according to claim 9, wherein the divalent linking group represented by $Y^1$ is one member selected from the group consisting of -NH-, -O-, -CO-O-, -O-CO-, -CO-NH-, -NH-CO-, -NH-CO-O-, -O-CO-NH-, -O-CO-O-, and -NH-CO-NH-.

**11.** The carbon nanomaterial according to claim 9 or 10, which is a carbon nanodiamond.

**12.** A pharmaceutical composition comprising the self-assembled nanocluster of claim 1,
the self-assembled nanocluster, together with a drug, being in the form of a composite.

Fig. 1

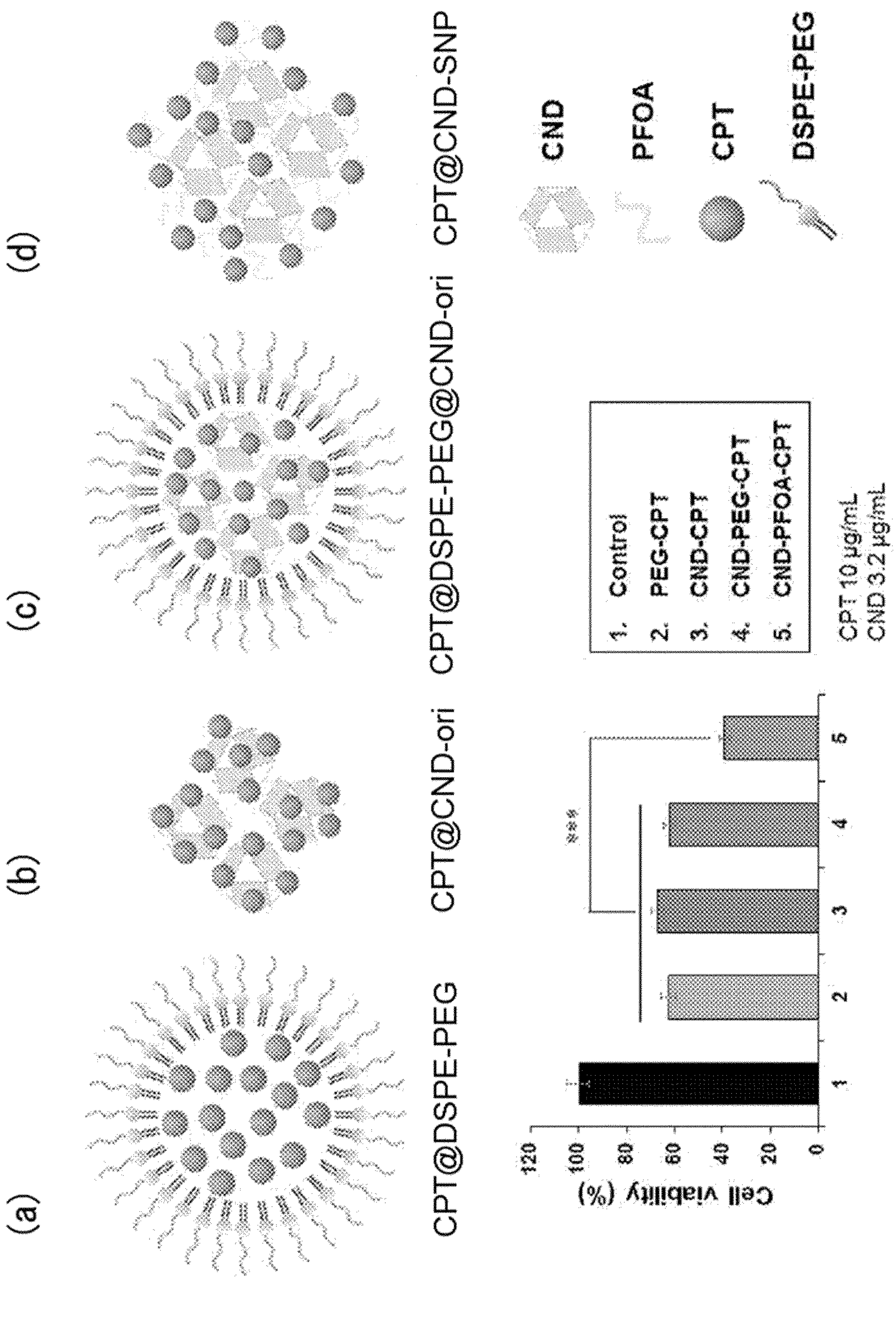
(a)
CPT@DSPE-PEG
(b)
CPT@CND-ori
(c)
CPT@DSPE-PEG@CND-ori
(d)
CPT@CND-SNP
CND
PFOA
CPT
DSPE-PEG
Cell viability (%)
0
20
40
60
80
100
120
1
2
3
4
5
***
1. Control
2. PEG-CPT
3. CND-CPT
4. CND-PEG-CPT
5. CND-PFOA-CPT
CPT 10 μg/mL
CND 3.2 μg/mL
(e)

Fig. 2

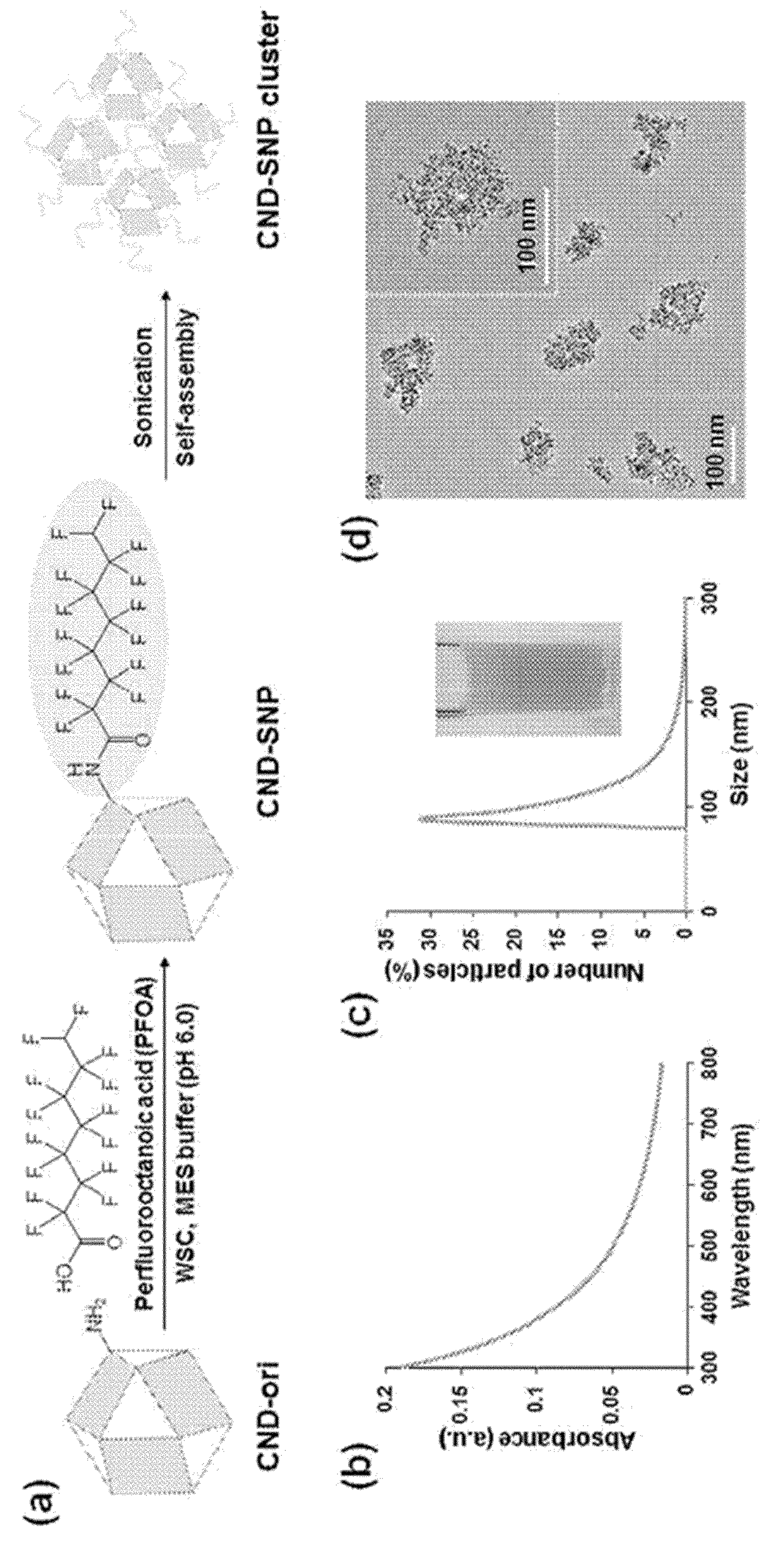
(a)
CND-ori
Perfluorooctanoicacid (PFOA)
WSC, MES buffer (pH 6.0)
CND-SNP
Sonication
Self-assembly
CND-SNP cluster
(b)
Absorbance (a.u.)
Wavelength (nm)
(c)
Number of particles (%)
Size (nm)
(d)
100 nm
100 nm

Fig. 3

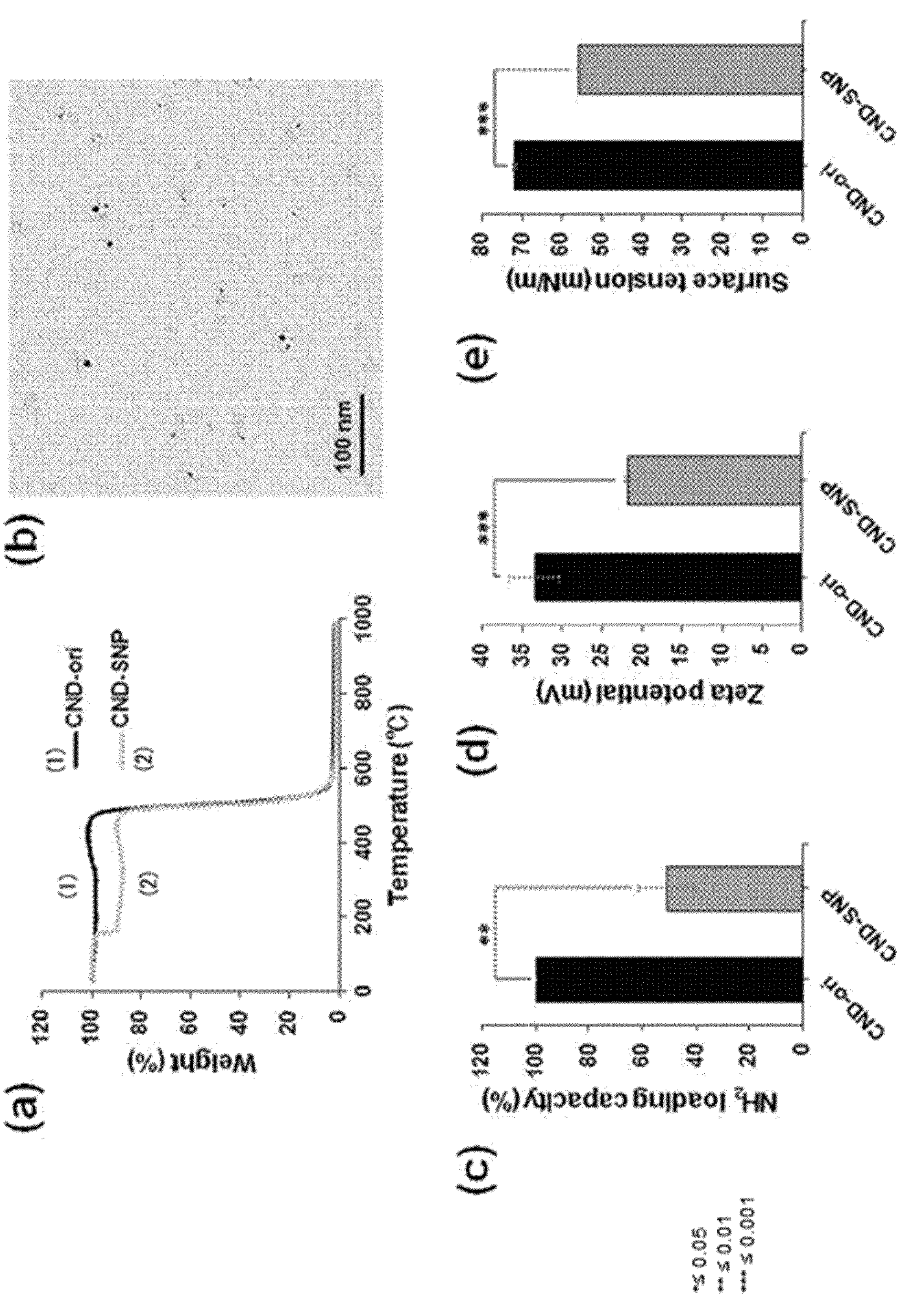
(a)
Weight (%)
Temperature (°C)
(1) CND-ori
(2) CND-SNP
(b)
100 nm
(c)
NH2 loading capacity (%)
CND-ori
CND-SNP
(d)
Zeta potential (mV)
(e)
Surface tension (mN/m)
* ≤ 0.05
** ≤ 0.01
*** ≤ 0.001

Fig. 4

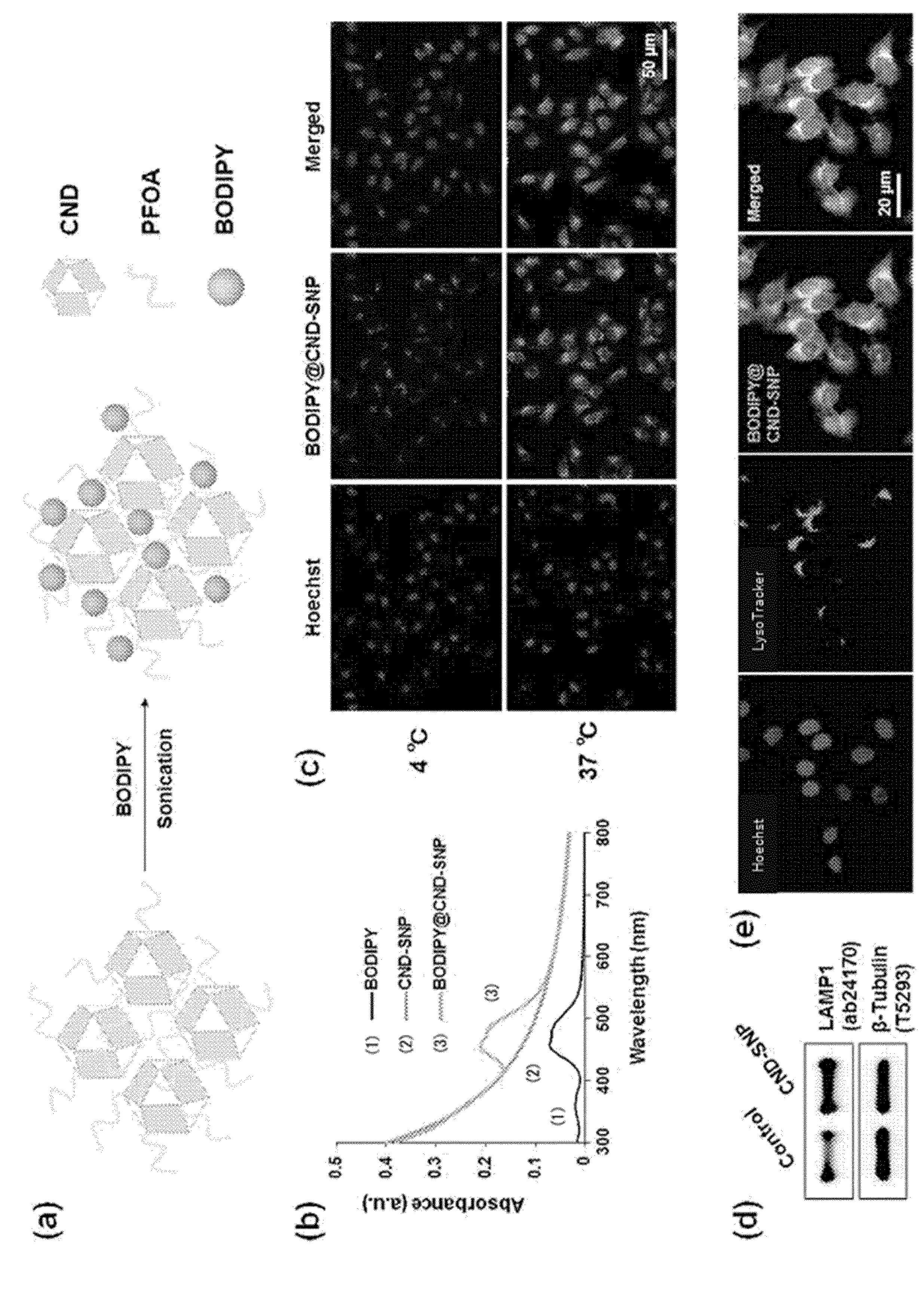

(a)
BODIPY
Sonication
CND
PFOA
BODIPY
(b)
Absorbance (a.u.)
Wavelength (nm)
(1) BODIPY
(2) CND-SNP
(3) BODIPY@CND-SNP
(c)
Hoechst
BODIPY@CND-SNP
Merged
4 °C
37 °C
50 μm
(d)
Control
CND-SNP
LAMP1 (ab24170)
β-Tubulin (T5293)
(e)
Hoechst
LysoTracker
BODIPY@ CND-SNP
Merged
20 μm

Fig. 5

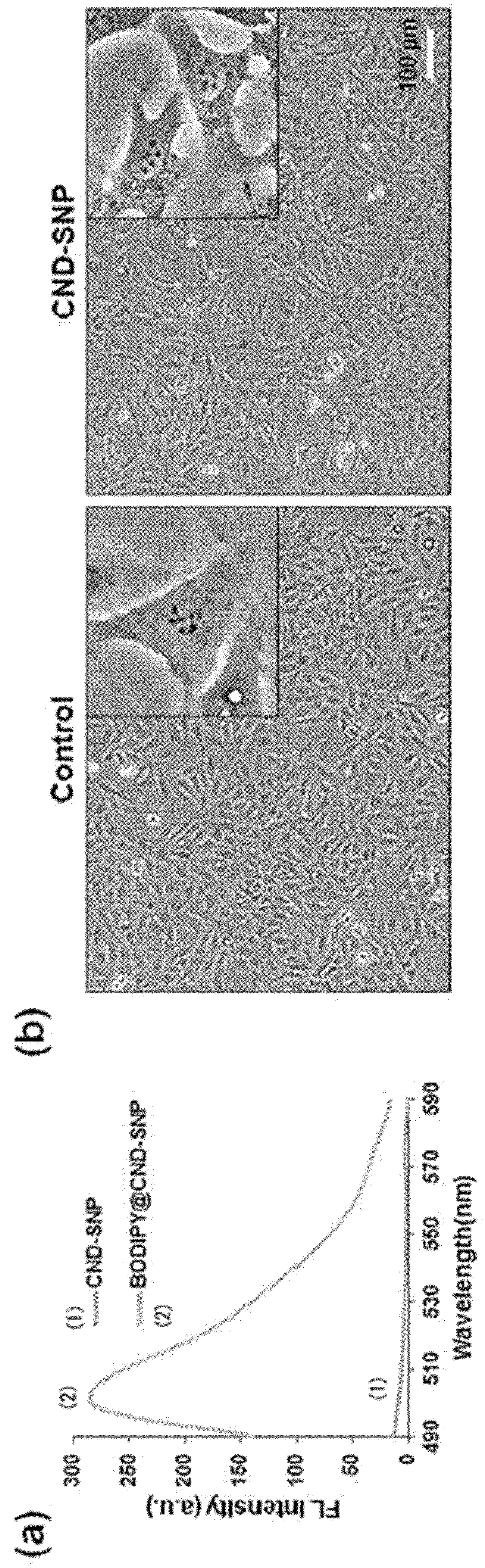
(a)
FL Intensity (a.u.)
Wavelength(nm)
(1) CND-SNP
BODIPY@CND-SNP (2)
(1)
(2)
0
50
100
150
200
250
300
490
510
530
550
570
590
(b)
Control
CND-SNP
100 μm

Fig. 6

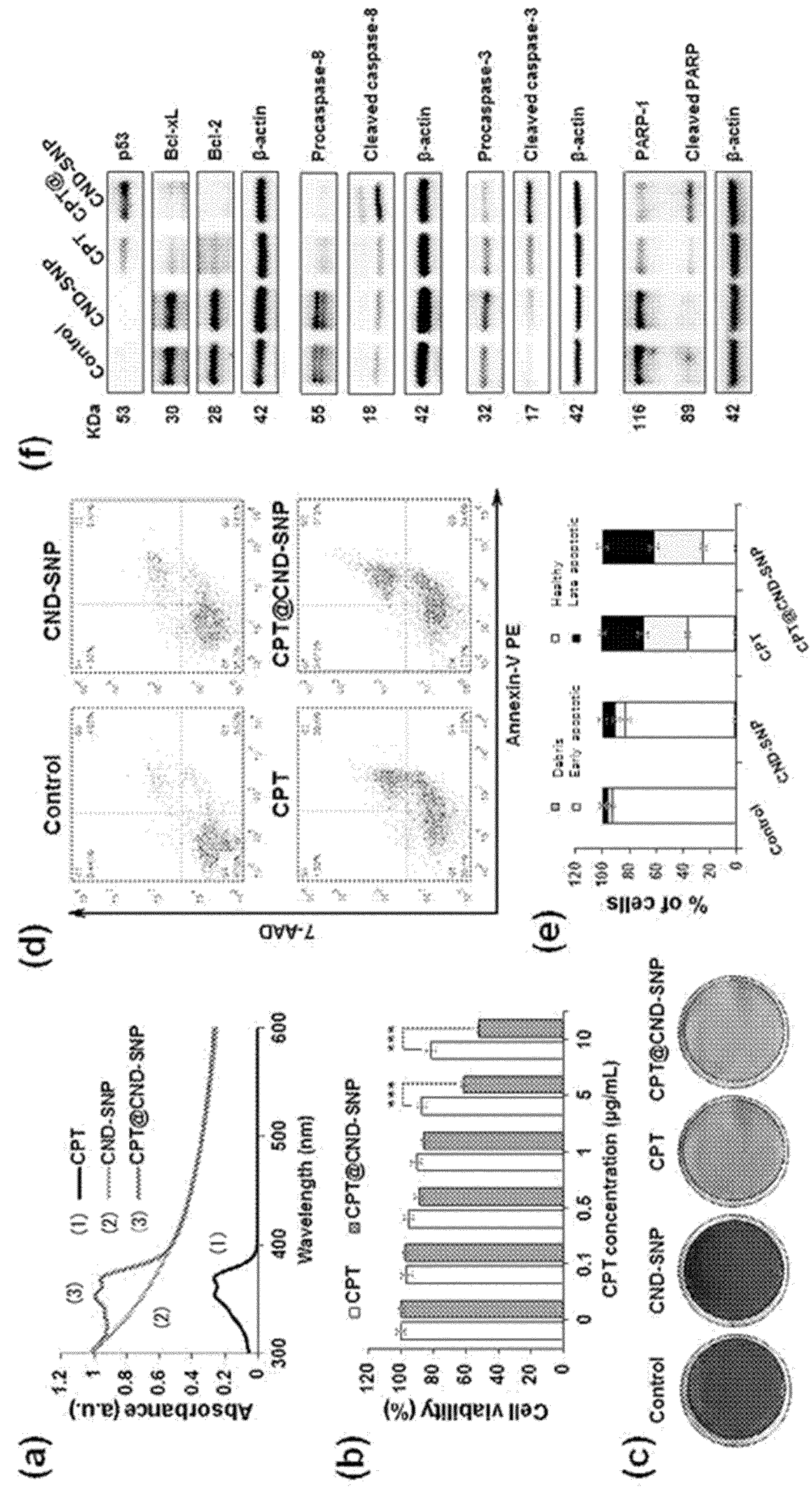
(a)
Absorbance (a.u.)
Wavelength (nm)
(1) CPT
(2) CND-SNP
(3) CPT@CND-SNP
(b)
Cell viability (%)
CPT concentration (μg/mL)
CPT
CPT@CND-SNP
(c)
Control
CND-SNP
CPT
CPT@CND-SNP
(d)
Control
CND-SNP
CPT
CPT@CND-SNP
7-AAD
Annexin-V PE
(e)
% of cells
Debris
Early apoptotic
Healthy
Late apoptotic
(f)
KDa
Control
CND-SNP
CPT
CPT@CND-SNP
p53
Bcl-xL
Bcl-2
β-actin
Procaspase-8
Cleaved caspase-8
Procaspase-3
Cleaved caspase-3
PARP-1
Cleaved PARP

Fig. 7

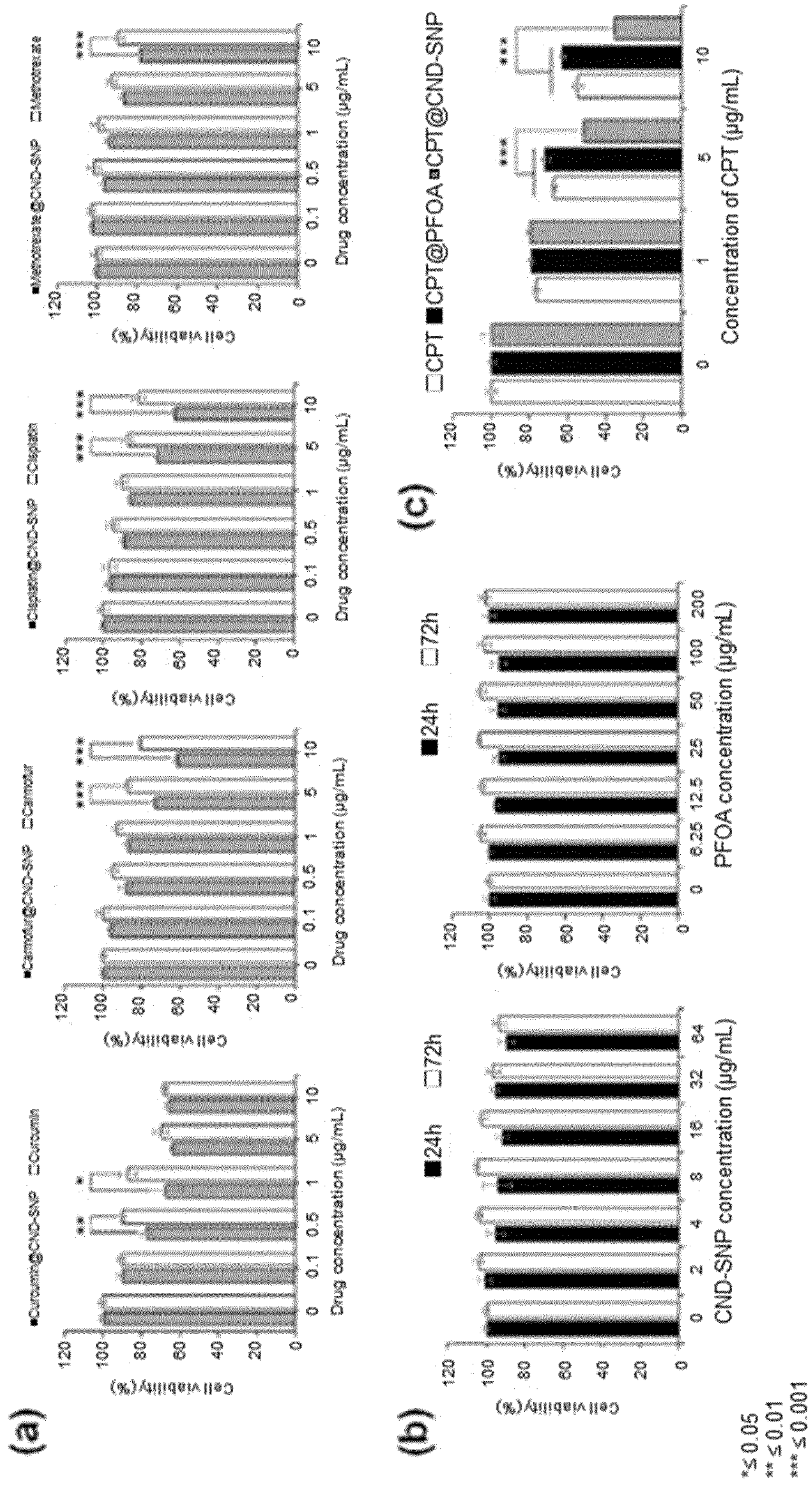
(a)
Curcumin@CND-SNP
Curcumin
Carmofur@CND-SNP
Carmofur
Cisplatin@CND-SNP
Cisplatin
Methotrexate@CND-SNP
Methotrexate
Cell viability (%)
Drug concentration (μg/mL)
(b)
24h
72h
CND-SNP concentration (μg/mL)
PFOA concentration (μg/mL)
(c)
CPT
CPT@PFOA
CPT@CND-SNP
Concentration of CPT (μg/mL)
*≤ 0.05
** ≤ 0.01
*** ≤ 0.001

Fig. 8

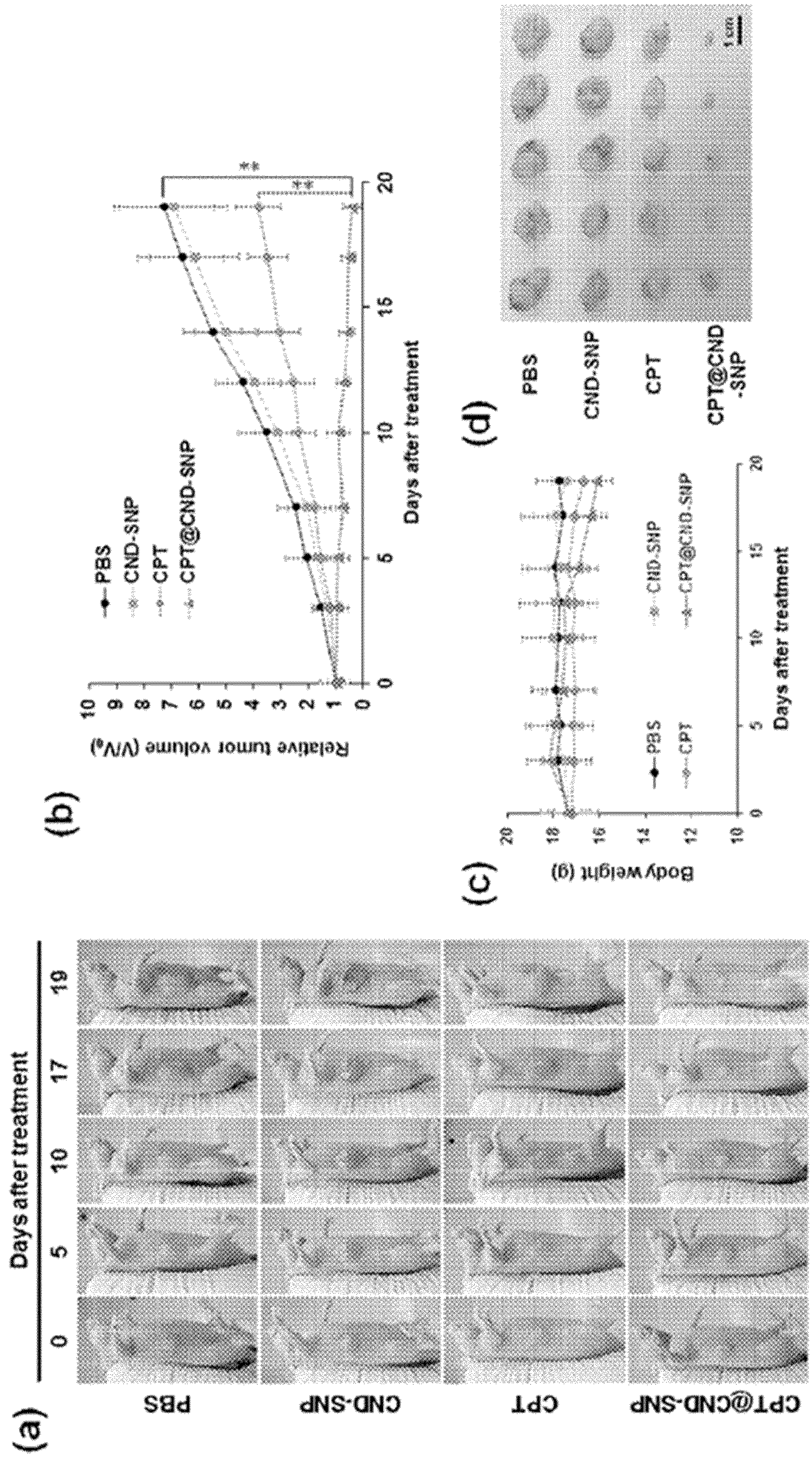
(a)
Days after treatment
0
5
10
17
19
PBS
CND-SNP
CPT
CPT@CND-SNP
(b)
Relative tumor volume (V/V₀)
Days after treatment
PBS
CND-SNP
CPT
CPT@CND-SNP
**
**
(c)
Body weight (g)
Days after treatment
PBS
CPT
CND-SNP
CPT@CND-SNP
(d)
PBS
CND-SNP
CPT
CPT@CND-SNP
1 cm

Fig. 9

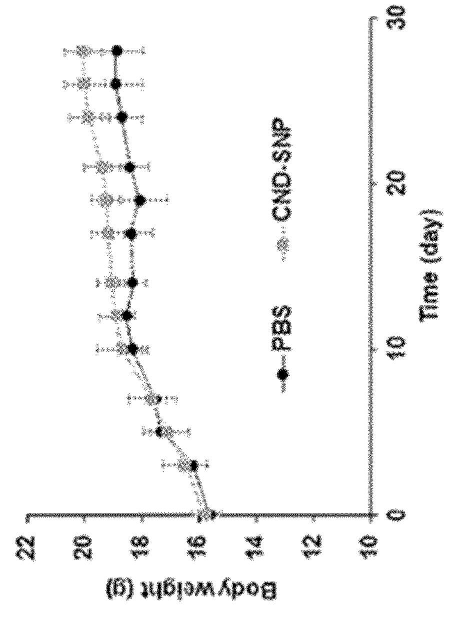

Fig. 10

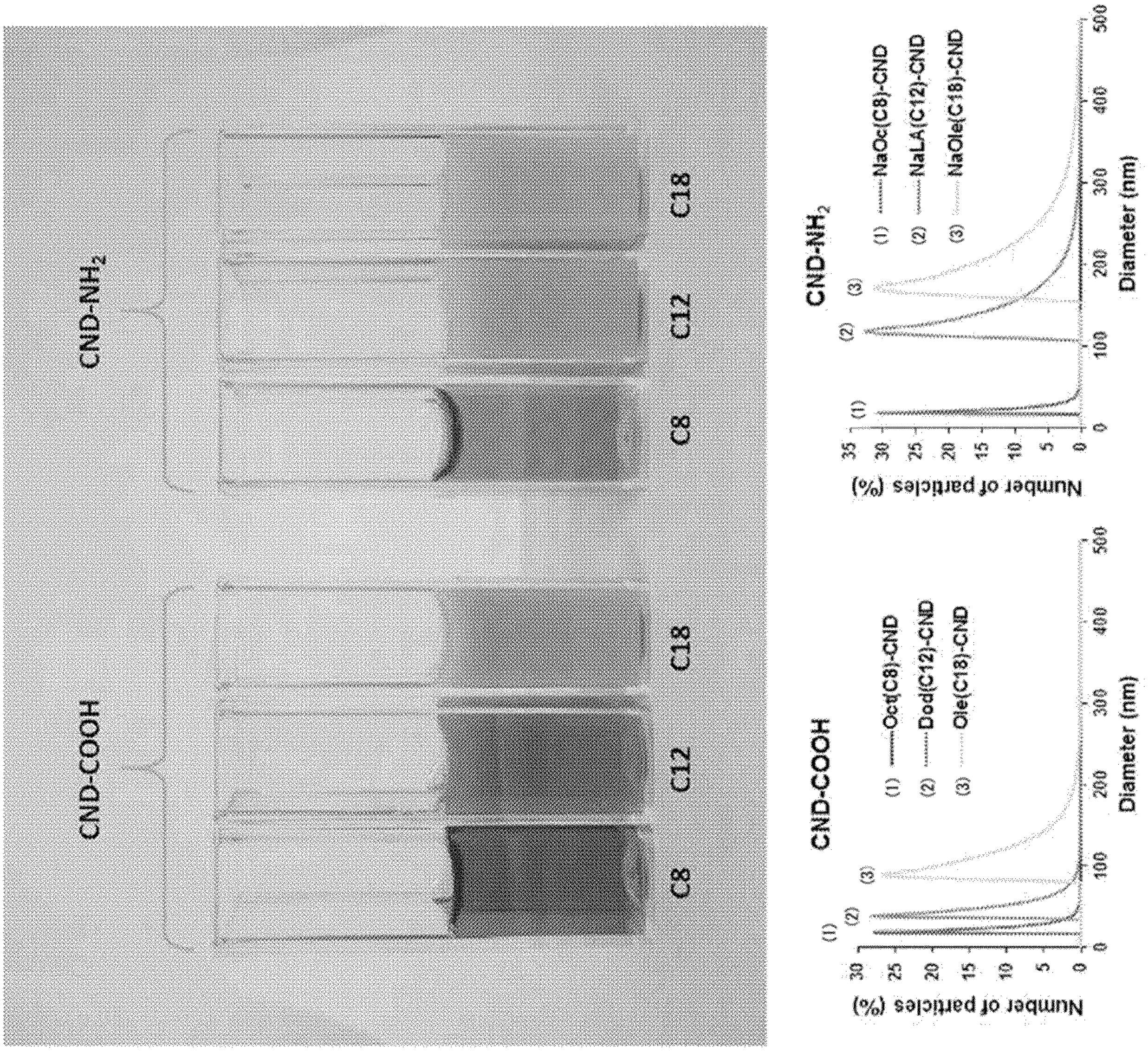
CND-COOH
CND-NH$_2$
C8
C12
C18
(1) Oct(C8)-CND
(2) Dod(C12)-CND
(3) Ole(C18)-CND
(1) NaOc(C8)-CND
(2) NaLA(C12)-CND
(3) NaOle(C18)-CND
Number of particles (%)
Diameter (nm)

## INTERNATIONAL SEARCH REPORT

International application No. PCT/JP2018/044300

**A. CLASSIFICATION OF SUBJECT MATTER**

Int.Cl. A61K47/02(2006.01)i, A61K9/14(2006.01)i, A61K31/4745(2006.01)i, A61K47/44(2017.01)i, C01B32/28(2017.01)i

According to International Patent Classification (IPC) or to both national classification and IPC

**B. FIELDS SEARCHED**

Minimum documentation searched (classification system followed by classification symbols)

Int.Cl. A61K47/02, A61K9/14, A61K31/4745, A61K47/44, C01B32/28

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

CAplus/REGISTRY/MEDLINE/EMBASE/BIOSIS(STN), JSTPlus/JMEDPlus/JST7580(JDreamIII), Science Direct, Japio-GPG/FX, PubMed

**C. DOCUMENTS CONSIDERED TO BE RELEVANT**

| Category* | Citation of document, with indication, where appropriate, of the relevant passages | Relevant to claim No. |
|---|---|---|
| X<br>Y | ALBRECHT, A. et al., Self-assembling hybrid diamond-biological quantum devices, New Journal of Physics, September 2014, vol. 16, pp. 1-7, S1-S28, abstract, formation of SP1 nanodiamond structures, fig. 1, ISSN: 1367-2630 | 1-7<br>8, 12 |
| X<br>Y | WANG, H. D. et al., Protein-modified nanodiamond particles for Layer-by-Layer assembly, Diamond and Related Materials, 29 June 2011, vol. 20, pp. 1193-1198, abstract, 2. Experimental, fig. 1, 2, ISSN: 0925-9635 | 1-7<br>8, 12 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 19.12.2018 | 15.01.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2018/044300

C (Continuation). DOCUMENTS CONSIDERED TO BE RELEVANT

| Category* | Citation of document, with indication, where appropriate, of the relevant passages | Relevant to claim No. |
|---|---|---|
| X | JP 2012-17225 A (VISION DEVELOPMENT CO., LTD.) 26 January 2012, claims 1-5, paragraphs [0061]-[0087] (Family: none) | 9-11 |
| Y | JP 2012-528197 A (NORTHWESTERN UNIVERSITY) 12 November 2012, claims 1-13, paragraphs [0004], [0005], [0016]-[0030] & WO 2010/138837 A2, claims 1-13, page 1, line 16 to page 2, line 27, page 11, line 30 to page 20, line 17 & US 2010/0305309 A1 & EP 2435360 A2 & CN 102459064 A | 8, 12 |
| A | JP 2017-186235 A (DAICEL CORPORATION) 12 October 2017 & WO 2017/170251 A1 & TW 201806857 A | 1-12 |
| A | JP 2008-303104 A (KOMATSU, Naoki) 18 December 2008 (Family: none) | 1-12 |
| A | JP 2009-119561 A (NATIONAL INSTITUTE OF ADVANCED INDUSTRIAL SCIENCE & TECHNOLOGY) 04 June 2009 (Family: none) | 1-12 |
| A | CN 104784703 A (BEIJING UNIVERSITY OF TECHNOLOGY) 22 July 2015 (Family: none) | 1-12 |
| A | KR 10-2017-0114265 A (THE CATHOLIC UNIV OF KOREA INDUSTRY-ACADEMIC COOP FOUND) 13 October 2017 (Family: none) | 1-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010202458 A
- JP 2017186234 A
- JP 2017186235 A
- JP 2010248023 A


### Non-patent literature cited in the description


- **BO GUAN et al.** *Small,* 2010, vol. 6, 1514-1519
- **LAURA MOORE et al.** *Adv. Mater.,* 2013, vol. 25, 3532-3541
- **A. KRUEGER.** *Chem. Eur. J.,* 2008, vol. 14, 1382-1390
- **Y. LIU.** *Chem. Mater.,* 2004, vol. 16, 3924-3930
- **A. KRUEGER.** *Langmuir,* 2008, vol. 24, 4200-4204
- **DENISOV S.A.** *IOP Conf. Series: Materials Science and Engineering,* 2010, vol. 16, 012005
- **A. KRUEGER.** *Advanced Functional Materials,* vol. 22 (5), 890-906
- **YU et al.** *Cell Death and Disease,* 2017, vol. 8, e2755